# EUROPEAN PATENT SPECIFICATION

(11) **EP 3 946 461 B1**
(45) Date of publication and mention of the grant of the patent: **15.01.2025**
(21) Application number: 20784479.6
(22) Date of filing: 03.04.2020
(51) Int. Cl.: A61K 47/61, A61P 1/00, C07J 17/00, A61K 35/74, A61K 31/573, A61K 31/58, A61K 31/618, C07H 15/00, C07H 15/20, C07J 5/00, C07J 71/00, A61K 35/742

(54) **XYLOGLUCAN-CONTAINING PRODRUGS AND METHODS OF MANUFACTURE AND USE THEREOF**
XYLOGLUCANHALTIGE PRODRUGS UND VERFAHREN ZU IHRER HERSTELLUNG UND IHRE VERWENDUNG
PROMÉDICAMENTS CONTENANT DU XYLOGLUCANE ET LEURS PROCÉDÉS DE FABRICATION ET D'UTILISATION

(30) Priority: 04.04.2019 GB 201904758
(43) Date of publication of application: 09.02.2022
(73) Proprietor: The University of British Columbia, Vancouver, British Columbia V6T 1Z3 (CA); His Majesty The King in Right of Canada, as Represented by The Minister of Agriculture and Agri-Food, Lethbridge, Alberta T1J 4B1 (CA); The Governors of the University of Alberta, Edmonton, AB T5J 4P6 (CA)
(72) Inventor: BRUMER, Harry, Vancouver, British Columbia V6S 1J9 (CA); HUI, Benjamin, Vancouver, British Columbia V6P 4J4 (CA); WANG, Chang Qing, Vancouver, British Columbia V5P 2V3 (CA); INGLIS, Gordon Douglas, Lethbridge, Alberta T1K 3C3 (CA); CLARKE, Sandra Theresa, Whitby, Ontario L1N 3T6 (CA); UWIERA, Richard Robert Ernest, Edmonton, Alberta T5T 5M9 (CA); ABBOTT, Dennis Wade, Lethbridge, Alberta T1K 7P2 (CA)
(74) Representative: Elend, Almut Susanne
(86) International application number: PCT/CA2020/050447
(87) International publication number: WO 2020/198878

(56) References cited:
- CA-A1- 3 023 797
- US-A1- 2007 173 461
- FRIEND D R ET AL: "Drug glycosides: potential prodrugs for colon-specific drug delivery", JOURNAL OF MEDICINAL CHEMISTRY, AMERICAN CHEMICAL SOCIETY, US, vol. 28, no. 1, 1 January 1985 (1985-01-01), pages 51 - 57, XP002745534, ISSN: 0022-2623, DOI: 10.1021/JM00379A012
- TAKAHASHI A ET AL: "PERCUTANEOUS ABSORPTION OF NON-STEROIDAL ANTI-INFLAMMATORY DRUGS FROM IN SITU GELLING XYLOGLUCAN FORMULATIONS IN RATS", INTERNATIONAL JOURNAL OF PHARMACEUTICS, ELSEVIER, NL, vol. 246, no. 1/02, 10 October 2002 (2002-10-10), pages 179 - 186, XP001154057, ISSN: 0378-5173, DOI: 10.1016/S0378-5173(02)00394-0
- ANINDYA ATSARINA LARASATI ET AL: "Xylan from Pineapple Stem Waste: a Potential Biopolymer for Colonic Targeting of Anti-inflammatory Agent Mesalamine", AAPS PHARMSCITECH, SPRINGER INTERNATIONAL PUBLISHING, CHAM, vol. 20, no. 3, 13 February 2019 (2019-02-13), pages 1 - 13, XP036702780, DOI: 10.1208/S12249-018-1205-Y
- XIE XUAN ET AL: "Targeted nanoparticles from xyloglucan-doxorubicin conjugate loaded with doxorubicin against drug resistance", RSC ADVANCES, vol. 6, no. 31, 10 March 2016 (2016-03-10), GB, pages 26137 - 26146, XP093032187, ISSN: 2046-2069, DOI: 10.1039/C6RA01779G
- BLIMAN ET AL.: "Enzymatically Activated Glyco-Prodrugs of Doxorubicin Synthesized by a Catalysis-Free Diels-Alder Reaction", BIOCONJUGATE CHEM., vol. 29, no. 7, 18 July 2018 (2018-07-18), pages 2370 - 81, XP055746475
- CAO ET AL.: "Intracellular Delivery of Mitomycin C with Targeted Polysaccharide Conjugates Against Multidrug Resistance", ANNALS OF BIOMEDICAL ENGINEERING, vol. 39, no. 9, 24 June 2011 (2011-06-24), pages 2456 - 65, XP019934423, DOI: 10.1007/s10439-011-0333-2

## Description

### CROSS-REFERENCE TO RELATED APPLICATIONS

The present application claims priority from United Kingdom Patent Application No. GB1904758.8, filed April 4, 2019.

### TECHNICAL FIELD

The present invention relates to synthetic prodrugs. In particular, the present invention relates to synthetic prodrugs comprising a chemically conjugated carbohydrate, and the use of the prodrugs for treatment of disorders, such as of enteric inflammatory diseases (EIDs).

### INTRODUCTION

The corticosteroids prednisolone (PRED) and budesonide (BUDE) have been used in the treatment of inflammatory bowel disease (IBD) for several decades and their pharmacology has been well studied. Their hydrophobicity allows relative ease of passage through cell membranes into the cytoplasm, where they bind to glucocorticoid receptors responsible for modulating genes controlling the inflammation process. The bound complexes may then be transported into the nucleus, where they induce the upregulation of anti-inflammatory protein expression.

5-aminosalicylic acid (5-ASA) is a member of the aminosalicylate class of IBD drugs that operate with a different mechanism hitherto unknown, although it is postulated that these molecules inhibit the function of cyclooxygenases and consequently the production of prostaglandins, which are responsible for the inflammation process in the colon.

These drugs suffer from nonspecific systemic uptake, with oral doses absorbed predominantly in the small intestine instead of the colon where the affects of IBD most commonly manifest. This negates much of the therapeutic effectiveness of these drugs, as they are most potent when applied directly to the site of inflammation. Furthermore, several undesirable side effects can arise as a result of the nonspecific uptake, including, for example, convulsions, hypertension, blood pressure increase, swelling of the stomach lining and an increased risk of stomach ulcers. Another drawback of these drugs is their low solubility, which leads to low bioavailabilities and poor pharmacodynamics.

In light of these problems, there is a continued interest in developing targeted drug delivery systems to the distal large intestine and colon. These systems may be considered broadly in two classes: formulations and prodrugs. In the former, the active drugs are compounded with various excipients and non-pharmaceutically active ingredients into pill or capsule forms having physical properties that allow a pH-dependent or time-delayed release. The latter involves chemically modifying the drug with a carrier molecule to inactivate it, and on reaching the target area, it is regenerated to its active form *in situ* spontaneously or by the enzymatic action of the local microflora.

Prodrugs of 5-aminosalicylic acid (5-ASA) have been widely reported. The principal example is sulfasalazine, an azo-based derivative of 5-ASA that was developed in the 1950s. On passage into the colon, sulfasalazine is metabolized into active 5-ASA by the azoreductase activity of the local microflora. While the prodrug is generally effective in treating IBD, the carrier molecule sulfapyridine released as the metabolic product gives rise to undesirable side effects which include malaise, drug hypersensitivity and impotency amongst others. A more recent 5-ASA prodrug in which it is chemically linked to taurine exhibited a markedly improved efficiency of colonic delivery where it is readily converted to 5-ASA.

Glycosylation is another strategy for prodrug synthesis and involves the chemical ligation of the drug to a carbohydrate carrier via *β*-glycosidic bonds. These conjugates are recognized as substrates by *β*-glycosidase enzymes produced by the gut microbiota and consequently broken down to release the active drug. Seminal work in this area was conducted by Friend and Chang who reported the synthesis of the *β*-glucosides of the corticosteroids prednisolone and dexamethasone. Oral doses were administered to rats and drug release was studied. The results showed increases in the delivery of the prodrugs to the cecum (pre-colon) as compared to the free drugs when administered as controls. No colonic delivery was detected however, and this was attributed to the limited testing time window before the animals were euthanized. Subsequently, a library of corticosteroid *β*-glycosides was prepared and kinetic studies were conducted on their hydrolysis *in vitro* by rat gastrointestinal tract contents. The results showed unequivocally higher rates of hydrolysis in the cecum compared to the stomach and small intestine. The therapeutic efficacy of these prodrugs was then tested on artificially induced IBD in different animal models with positive results.

*β*-glucosides of several 5-ASA derivatives have also been reported and demonstrated anti-inflammatory effects in the same order of magnitude as the parent drug on chemically induced ulceritis in mice. No studies on the site specificity of drug release were conducted. More recently, the *β*-gluco- and galactosides of 5-ASA have been reported with improved solubility, pharmacodynamics and specificity of colonic release in rat models. US2007/173461A1 discloses β-O-glycoside prodrugs of 5-aminosalicylic acid (5-amino-2(β-D-galactopyranosyloxy)benzoic acid, 5-amino-2-(β-D-glucopyranosyloxy)benzoic acid) as therapeutic agents for ulcerative colitis and methods of their preparation.

Friend D.R., 1985, discloses (abstract, scheme 1): β-D-glucosides and galactosides of dexamethasone, prednisolone and their applications in colon-specific drug delivery. In spite of advances in targeted drug delivery as described above, to date there is no known method for the delivery of an anti-inflammatory therapeutic agent to the colon without hydrolysis or metabolism in the proximal gastrointestinal tract, that causes minimal side effects and that can be administered over the long term.

The above information is provided for the purpose of making known information believed by the applicant to be of possible relevance to the present invention. No admission is necessarily intended, nor should be construed, that any of the preceding information constitutes prior art against the present invention.

### SUMMARY

An object of the present application is to overcome the drawbacks associated with current colonic drug delivery by providing a therapeutic agent that is resistant to hydrolysis or metabolism in the proximal gastrointestinal tract (stomach, small and large intestines), but allows the release of an active drug, for example, for IBD treatment, in the colon, and that is safe for long term administration.

In accordance with an aspect of the present application, there is provided xyloglucan-containing prodrugs, referred to herein as glyco-caged prodrugs, and methods of manufacture and use thereof.

In accordance with one aspect of the present application, there is provided a prodrug compound of formula (I) or (II)

xyloglucan - *β* - O - R^{D} (I)

xyloglucan - *β* - O - L^{SI} - R^{D} (II)

wherein *β* - O represents a *β*-O-glycosidic bond, R^{D} is a drug moiety and L^{SI} is a self-immolative linker, or a pharmaceutically acceptable salt or solvate thereof, wherein cleavage of the *β*-O-glycosidic bond connecting the xyloglucan to R^{D} or L^{SI} results in formation of a drug from the drug moiety.

In accordance with another aspect, there is provided a composition comprising a prodrug compound of formula (I) or (II).

In accordance with another aspect, there is provided a prodrug compound of formula (I) or (II), or a composition comprising a prodrug compound of formula (I) or (II), for use in a method of treating a gastrointestinal disorder by administering the prodrug or the composition to a subject in need thereof.

In accordance with another aspect, there is provided a method for producing a prodrug compound of formula (I)

xyloglucan - *β* - O - R^{D} (I)

wherein - O represents a *β*-O-glycosidic bond and R^{D} is a drug moiety, or a pharmaceutically acceptable salt or solvate thereof, said method comprising: (a) protecting the hydroxyl groups of a xyloglucan to form a protected xyloglucan; (b) performing a glycosylation reaction to form a glycosidic bond between the protected xyloglucan and a drug or a drug derivative; and (c) deprotecting the product of step (b).

In accordance with another aspect, there is provided a method for producing a prodrug compound of formula (II)

xyloglucan - *β* - O - L^{SI} - R^{D} (II)

wherein *β* - O represents a β-O-glycosidic bond, R^{D} is a drug moiety and L^{SI} is a self-immolative linker moiety, or a pharmaceutically acceptable salt or solvate thereof, said method comprising: (a) protecting the hydroxyl groups of a xyloglucan to form a protected xyloglucan; (b) reacting the protected xyloglucan to form a glycosyl halide; (c) performing a glycosylation reaction to form a glycosidic bond between the protected xyloglucan and a linker molecule to form a compound of formula (III)

xyloglucan - *β* - O - L^{SI} (III) ;

and (d) attaching a drug or a drug derivative to L^{SI} of the compound of formula (III).

### BRIEF DESCRIPTION OF FIGURES

For a better understanding of the application as described herein, as well as other aspects and further features thereof, reference is made to the following description which is to be used in conjunction with the accompanying drawings, where:
Figure 1 schematically depicts a release of a pharmaceutically active drug from a glyco-caged prodrug according to one embodiment described herein;
Figures 2A-D depict examples of small molecule and peptide drugs that can be incorporated in a glyco-caged prodrug in accordance with certain embodiments (A 5-aminosalicylic acid, B corticosteroids, C non-steroidal antiflammatory agents, and D therapeutic peptides), where the arrows indicate groups suitable for functionalization to form a glyco-caged prodrug;
Figures 3A-B schematically depict the function of self-immolative linkers in the glyco-caged prodrugs according to certain embodiments, where Figure 3A illustrates the general concept and Figure 3B illustrates specific examples for a amine or alcohol containing drugs;
Figure 4 graphically depicts Michaelis-Menten kinetic data for the hydrolysis of XXXG-5ASAME prodrug to release the active drug, SASAME;
Figure 5 graphically depicts Michaelis-Menten kinetic data for the hydrolysis of XXXG-PRED prodrug to release the active drug, prednisolone;
Figure 6 graphically depicts Michaelis-Menten kinetic data for the hydrolysis of XXXG-BUDE prodrug to release the active drug, budesonide;
Figure 7 graphically depicts Michaelis-Menten kinetic data for the hydrolysis of XXXG-DEXA prodrug to release the active drug, budesonide;
Figure 8 graphically depicts total weight loss observed in mice during treatment with drugs or glyco-caged drugs, where the weight loss was not significant between treatments or controls and animals remained at a healthy weight. *n* = 2 for each treatment group (inoculated with both *Bacteroides ovatus* and *Citrobacter rodentium*) and *n* = 4 for control 1 (germ-free) and control 2 (*C. rodentium* inoculated only);
Figures 9A-E are photographs of gross visual observations of the murine abdomen (A no steroid treatment; B dexamethasone; C XXXG-DEXA; D prednisolone; E XXXG-PRED), where less cecal and colonic thickening was observed in the XXXG-DEXA treated mice than the dexamethasone treated mice and both prednisolone and XXXG-PRED displayed more blood in cecal area than ± caged dexamethasone treated mice;
Figures 10A-G are images from histopathological analysis of the cecum three days following steroid treatment (A Germ-free control; B *Citrobacter rodentium* infection only, no steroid treatment; C *Bacteroides ovatus* and *C. rodentium* infection, no steroid treatment; D dexamethasone treated; E XXXG-DEXA treated; F prednisolone treated; G XXXG-PRED treated; D-G were inoculated with both *B. ovatus* and *C. rodentium),* where the oval on Figure 6B is highlighting an area of severe inflammation incited by *C. rodentium;*
Figures 11A-G are images from histopathological analysis of the proximal colon three days following steroid treatment (A Germ-free control; B *Citrobacter rodentium* infection only, no steroid treatment; C *Bacteroides ovatus* and *C. rodentium* infection, no steroid treatment; D dexamethasone treated; E XXXG-DEXA treated; F prednisolone treated; G XXXG-PRED treated; D-G were inoculated with both *B. ovatus* and *C. rodentium),* where the oval Figure 7D is highlighting an area of severe inflammation incited by *C. rodentium*;
Figures 12A-G are images from histopathological analysis of the distal colon three days following steroid treatment (A Germ-free control; B *Citrobacter rodentium* infection only, no steroid treatment; C *Bacteroides ovatus* and *C. rodentium* infection, no steroid treatment; D dexamethasone treated; E XXXG-DEXA treated; F prednisolone treated; G XXXG-PRED treated; D-G were inoculated with both *B. ovatus* and *C. rodentium),* where the oval on Figure 8B is highlighting an area of severe inflammation incited by *C. rodentium*;
Figures 13A-D graphically depict cytokine concentrations in cecal tissues in mice with inflammation incited by *Citrobacter rodentium* (five days post-inoculation), where the mice were administered uncaged ("free") or caged ("conjugated") dexamethasone or prednisolone (A IL-1β (pg/g); B IL-6 (pg/g); C TNF-α (pg/g); D IL-22 (pg/g); *n* = 2 for each treatment; note: IL-1β, IL-6, and TNF-α are highly pro-inflammatory cytokines, and IL-22 is an anti-inflammatory cytokine);
Figures 14A-C graphically depict cytokine concentrations in colonic tissues in mice with inflammation incited by *Citrobacter rodentium* (five days post-inoculation), where mice were administered uncaged ("free") or caged ("conjugated") dexamethasone or prednisolone (A IL-6 (pg/g); B IL-22 (pg/g); C TNF-α (pg/g); *n* = 2 for each treatment; note: IL-6 and TNF-α are highly pro-inflammatory cytokines, and IL-22 is an anti-inflammatory cytokine);
Figures 15A-B graphically depict the endo-xyloglucanase activity of strains of bacteria isolated from pig intestines in metabolism (decaging) of XXXG-resorufin (15A shows results from lysed cells and 15B shows results from whole cells, each grown on glucose or xyloglucan as the carbon source);
Figure 16 graphically depicts the distribution of *Bacteriodes* in the gastro-intestinal tract of pigs;
Figure 17 graphically depicts results from an *ex vivo* study of endo-xyloglucanse activity in decaging XXXG-resorufin in a pig colon bioreactor;
Figure 18 graphically depicts the activity of isolates of *Bacteroides ovatus* from chicken intestines in decaging XXXG-resorufin;
Figures 19A-B illustrate the presence of xyloglucanase activity in feces from mice, where Figure 19A shows xyloglucanase activity from bacteria cultured from feces of control mice (XyG-) and from mice fed xyloglucan enriched diets (XyG+), and Figure 19B graphically depicts the ability of the bacteria cultured from feces of control mice (XyG-) and from mice fed xyloglucan enriched diets (XyG+) in decaging XXXG-resorufin;
Figure 20 schematically depicts a murine study design and timeline for *Citrobacter rodentium*-induced colitis treatment; and
Figures 21A-F are images from histopathological analysis of the distal colon one day following steroid treatment (A Germ-free control; B *Bacteroides ovatus* and *C. rodentium* infection, no steroid treatment; C prednisolone treated; D XXXG-PRED treated; E dexamethasone treated; F XXXG-DEXA treated; C-F were inoculated with both *B. ovatus* and *C. rodentium*).

### DETAILED DESCRIPTION

### Definitions

Unless defined otherwise, all technical and scientific terms used herein have the same meaning as commonly understood by one of ordinary skill in the art to which this invention belongs.

As used in the specification and claims, the singular forms "a", "an" and "the" include plural references unless the context clearly dictates otherwise.

The term "comprising" as used herein will be understood to mean that the list following is non-exhaustive and may or may not include any other additional suitable items, for example one or more further feature(s), component(s) and/or ingredient(s) as appropriate.

As used herein, the term "BoGH5A" refers to glycoside hydrolase 5A, which is an endo-xyloglucanase enzyme, produced by *Bacteroides ovatus* that recognizes and digests xyloglucan by cleaving internal glycosidic bonds at the non-substituted glucose moiety of the xyloglucan backbone.

As used herein, the term "enteric" indicates a relation to all or a portion of the intestine or the intestinal tract, which includes the small intestine (i.e., the duodenum, the jejunum, and the ileum) and the large intestine (i.e., the cecum, the colon and the rectum).

As used herein, the terms "IBD" and "inflammatory bowel disease" are used interchangeably to describe a group of inflammatory conditions of the colon and small intestine. IBD includes, but is not limited to, the conditions of ulcerative colitis (UC) and Crohn's disease (CD). Both conditions are characterized by varying degrees of erosive inflammation and ulceration predominantly in the distal large intestine and colon.

As used herein, the term "in need thereof' is used to refer to a judgment made by a physician or other caregiver (e.g., a veterinarian) that a subject requires or will benefit from treatment or preventative care. This judgment is made based on a variety of factors that are in the realm of the physician's or caregiver's expertise.

As used herein, "pharmaceutically acceptable" means approved or approvable by a regulatory agency of a federal or a state/provincial government or the corresponding agency in countries other than the United States or Canada, or that is listed in the U.S. Pharmacopoeia or other generally recognized pharmacopoeia for use in animals, including in humans.

As used herein, the term "prodrug" will be understood to refer to a compound that, following administration to a subject, is metabolized into a pharmacologically active drug. The term "decaged" is used herein to reference the metabolism of glyco-caged prodrugs to release pharmacologically acid drug.

As used herein, the term "subject" refers to a human or a non-human animal (e.g., a mammal or a bird).

The present application provides prodrugs that are designed to treat, prevent or ameliorate the symptoms of enteric diseases and disorders, such as inflammatory bowel disease (IBD). The prodrugs of the present application comprise a drug portion and a carbohydrate portion that is cleaved following administration to a subject to release the pharmaceutically active drug. In particular, the prodrugs of the present application target release of an active drug in the intestinal tract through the incorporation of a xyloglucan moiety, which is specifically hydrolyzed by symbiotic bacteria found in the lower gastrointestinal tract. The prodrugs provided herein are referred to as "glyco-caged prodrugs" or "caged prodrugs", due to the incorporation of the xyloglucan moiety.

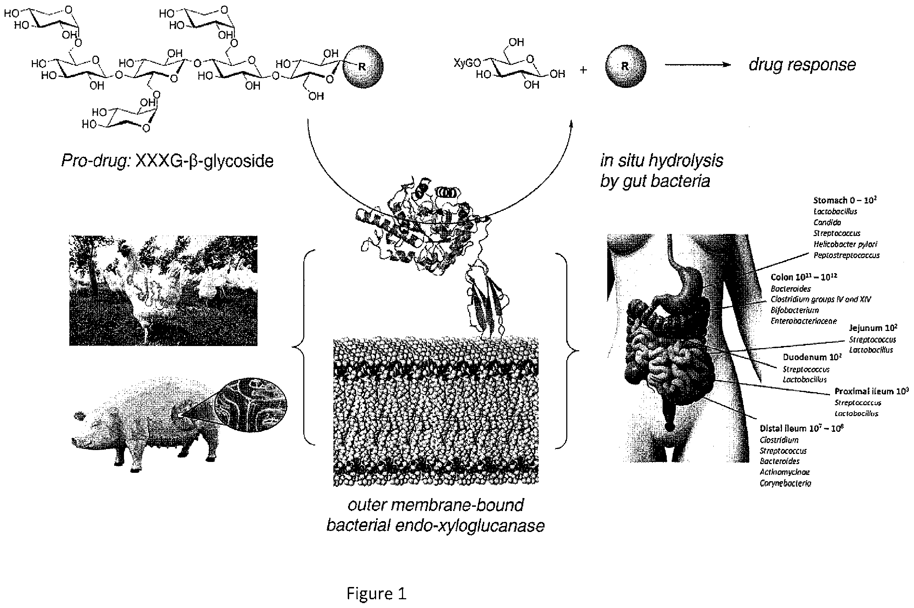

An example of the general process of pharmacologically active drug release from a glyco-caged prodrug is depicted in Figure 1. As shown in Figure 1, the glyco-caged prodrug comprising an XXXG xyloglucan, is hydrolysed *in situ* by a bacterial endo-xyloglucanase that is bound to the outer membrane of native bacteria in the gut.

The glyco-caged prodrugs of the present application are specifically hydrolysed by endo-xyloglucanases, for example those found in *Bacteroidetes,* such as *Bacteroides ovatus* or *Bacteroides uniformis,* for example those found in *Bacteroidetes,* such as *Bacteroides* species having a Xyloglucan Utilization Locus that is homologous to that found in *Bacteroides ovatus.* For example, the glyco-caged prodrugs can be specifically hydrolysed by endo-xyloglucanases found in *Bacteroides ovatus, Bacteroides uniformis, Bacteroides cellulosyliticus, Bacteroides faecis*, *Bacteroides xylanisolvens, Bacteroides fluxus, Bacteroides coprocola* and *Bacteroides salanitronis,* which are found in the native flora of the subject's lower gastrointestinal tract, including in mammalian (e.g., human or swine) or avian lower gastrointestinal tract. Other bacteria having an endo-xyloglucanase that can effectively hydrolyse xyloglucans, and therefore, the glyco-caged prodrugs of the present application can include, but are not limited to, *Bacillus parlicheniformis, Paeniclostridium sordellii, Parabacteroides distansonis, Prevotella* sp., *Salmonella enterica, Streptococcus gallolyticus* (e.g., subsp. *gallolyticus*). Consequently, the glyco-caged prodrugs can be effectively targeted for release of the pharmaceutically active drug in the lower gastrointestinal tract of a subject, such as a mammalian subject (e.g., a human or a swine) or an avian subject (e.g., a chicken).

### Glyco-Cased Prodrugs

In accordance with certain exemplary embodiments there is provided a glyco-caged prodrug compound of formula (I) or (II)

xyloglucan - *β* - O - R^{D} (I)

xyloglucan - *β* - O - L^{SI} - R^{D} (II)

wherein *β* - O represents a *β*-glycosidic bond, R^{D} is a drug moiety and L^{SI} is a self-immolative linker moiety, or a pharmaceutically acceptable salt or solvate thereof, wherein cleavage of the *β*-glycosidic bond connecting the xyloglucan to R^{D} or L^{SI} results in formation and release of a drug from the drug moiety.

Xyloglucan is the term used to refer to a class of linear polysaccharides consisting of (β1→4)-linked d-glucan substituted with xylose. In particular, the backbone of xyloglucan is (β1→4)-linked d-glucan, which is the same as cellulose, and three of the four glucose residues are substituted with a-d-xylose at the O6 position. This is the common structure for all the xyloglucan oligosaccharides, but additional residues are attached to xylose, depending on the source of xyloglucan. Typically, xyloglucans are not homopolymers, but a mix of these oligosaccharide substructures. A description of xyloglucan side chain types is provided in Pauly M. and Keegstra K. (2016) "Biosynthesis of the Plant Cell Wall Matrix Polysaccharide Xyloglucan." Ann. Rev. Plant Bio., Vol 67: 235 - 259. The structures of four types of xyloglucan oligosaccharides, namely XXXG, XLXG, XLLG and XXLG types are shown below:

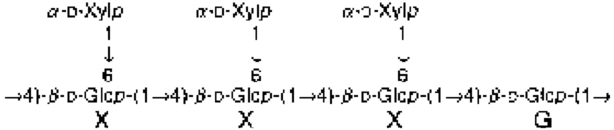

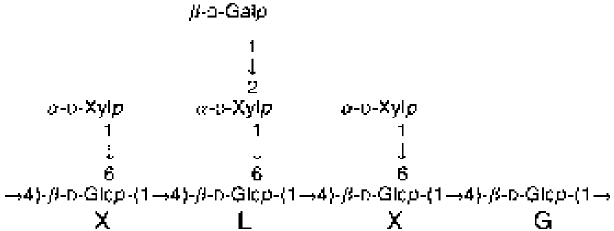

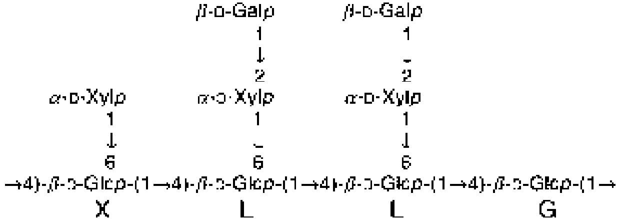

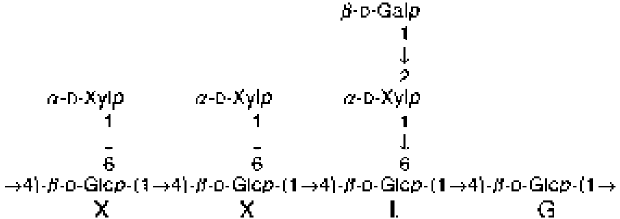

The xyloglucan portion of the glyco-caged prodrug of the present application can be any xyloglucan or a xyloglucan oligosaccharide. In certain embodiments, the glyco-caged prodrug comprises a xyloglucan that is or comprises an XXXG-, XXLG-, XLXG-, or XLLG-type xyloglucan, or a mixture of two or more of XXXG-, XXLG-, XLXG-, or XLLG-type xyloglucans. In a specific embodiment, the xyloglucan is a xyloglucan oligosaccharide, for example, an XXXG-type xyloglucan oligosaccharide.

Xyloglucan can be obtained from plant material, such as described in, for example, Hoffman, M. et al. (2005) "Structural analysis of xyloglucans in the primary cell walls of plants in the subclass Asteridae." Carbohydr. Res., Vol 340 (11): 1826 - 1840, and Buckeridge M.S. et al. (1997) "A new family of oligosaccharides from the xyloglucan of Hymenaea courbaril L. (Leguminosae) cotyledons." Carbohydr. Res., Vol 303 (2): 233 - 237. In one specific example, the XXXG xyloglucan is prepared from xyloglucan extracted from tamarind seed kernels. The tamarind XyG, which is composed of a mix of oligosaccharide substructures, is extracted. then liberated with an endo-xyloglucanase, and treated to remove the galactose on the XLLG, XXLG, and XLXG variants and treated with galactosidase to reduce the mixture to XXXG.

The drug moiety of the glyco-caged prodrug is selected based on the particular disorder condition, or disease to be treated. The attachment of the drug moiety to the xyloglucan moiety will vary depending on the chemical structure of the drug. However, in each case the structure of the prodrug comprises a *β*-glycosidic bond that is cleavable by endo-xyloglucanases, such as those found in *Bacteroidetes.*

The drug moiety can be based on, for example, a small molecule drug or a therapeutic peptide. Examples of suitable small molecule drugs include, but are not limited to: aminosalicylates, such as, 5-aminosalicylic acid (5-ASA, or mesalazine) or methyl mesalazine; corticosteroids, such as, prednisolone, budesonide, dexamethasone or isofluprednone; non-steroidal anti-inflammatory drugs, such as, meloxicam, flunixin or ketoprofen, or cannabinoids, endo-cannabinoids, or analogues thereof. The drug moiety can also be a natural product with anti-inflammatory and/or anti-micorbial properties (e.g., thymol, methol and the like). Non-limiting examples of therapeutic peptides that can be incorporated in a glyco-caged prodrug are larazotide and cathelicidin KR-12.

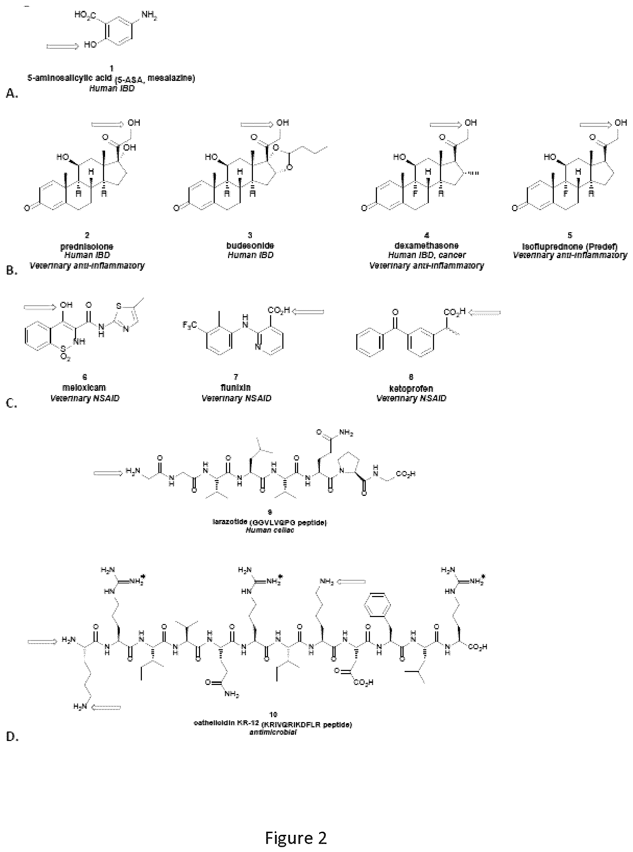

Figure 2 provides examples of small molecule and peptide drugs that can be used in the treatment of enteric inflammatory diseases and that can be incorporated as the drug moiety of a glyco-caged prodrug according to the present application. The arrows in Figure 2 indicate groups that are suitable for functionalization to form the corresponding glyco-caged prodrug.

In specific examples the glyco-caged prodrug is XXXG-*β*-O-dexamethasone (XXXG-DEXA), XXXG-*β-*O-prednisolone (XXXG-PRED), XXXG-*β*-O-budesonide (BUDE), XXXG-*β*-O-mesalazineME (XXXG-5ASAME), XXXG-*β*-O-mesalazine (XXXG-SASA), or XXXG- *β*-O- SIL mesalazine (XXXG-SIL-SASA).

The diversity in the structure of drugs that are suitable for the treatment of enteric inflammatory diseases and disorders requires the use of various synthetic strategies for attaching the drugs to the xyloglucan moiety. In some embodiments, the drug is attached directly to the xyloglucan moiety via a *β*-glycosidic bond. In other embodiments the drug is attached to the xyloglucan moiety via a self-immolative linker (SIL).

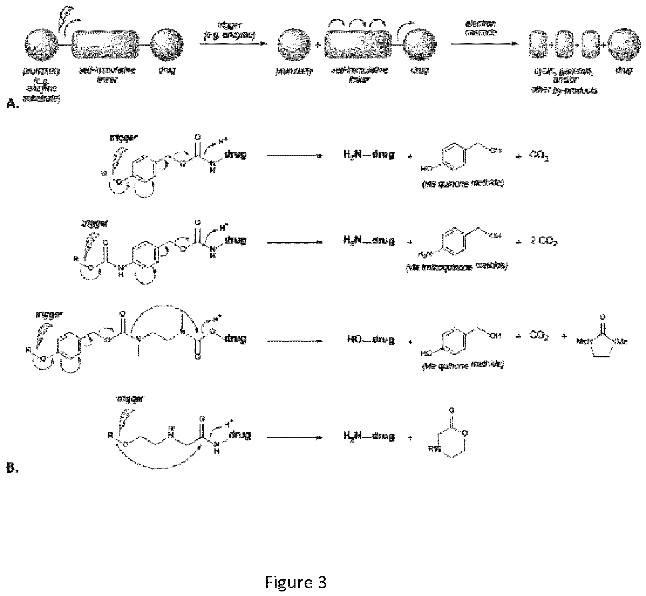

A self-immolative linker, or SIL, useful in the glyco-caged prodrug of the present application, includes a *β*-glycosidic bond that is susceptible to hydrolysis by endo-xyloglucanases found in *Bacteroidetes,* such as *Bacteroides ovatus* or *Bacteroides uniformis.* The hydrolysis triggers a reaction, or a cascade of reactions, resulting in the break down of the self-immolative linker and release of the pharmaceutically active drug moiety. This is illustrated schematically in Figure 3.

As illustrated in Figure 3A, the self-immolative linker based glyco-caged prodrug comprises a "promoiety," which in this case is a xyloglucan. The xyloglucan is attached to the self-immolative linker via a *β*-glycosidic bond. Cleavage of this bond by an endo-xyloglucanase is the trigger (shown by the lightening bolt in Figure 3A), that initiates the break down of the self-immolative linker and subsequent release of the pharmaceutically active drug. Figure 3B provides non-limiting examples of self-immolative linkers that can be used for hydroxyl- and amine-containing drugs. These linkers can be readily adapted to enable the conjugation of carboxylic acid-containing drugs (e.g., as analogous benzyl esters).

### Synthesis of Glyco-Caged Prodrugs

### 1. Direct Conjugation to Xyloglucan

Provided herein is a method for synthesizing a prodrug compound of formula (I)

xyloglucan - *β* - O - R^{D} (I)

wherein *β* - O represents a *β*-glycosidic bond, R^{D} is a drug moiety, or a pharmaceutically acceptable salt or solvate thereof. The method generally comprises: (a) protecting the hydroxyl groups of a xyloglucan to form a protected xyloglucan; (b) performing a glycosylation reaction to form a glycosidic bond between the protected xyloglucan and a drug or a drug derivative; and (c) deprotecting the product of step (b). Optionally, the step of forming the glycosidic bond between the protected xyloglucan and a drug or drug derivative comprises first reacting the protected xyloglucan to form a glycosyl halide and then performing a substitution reaction with an alcohol-containing drug or drug derivative and the glycosyl halide.

The synthetic scheme provided below summarizes examples of chemoenzmatic methods and chemical methods of synthesizing a prodrug compound of formula (I).

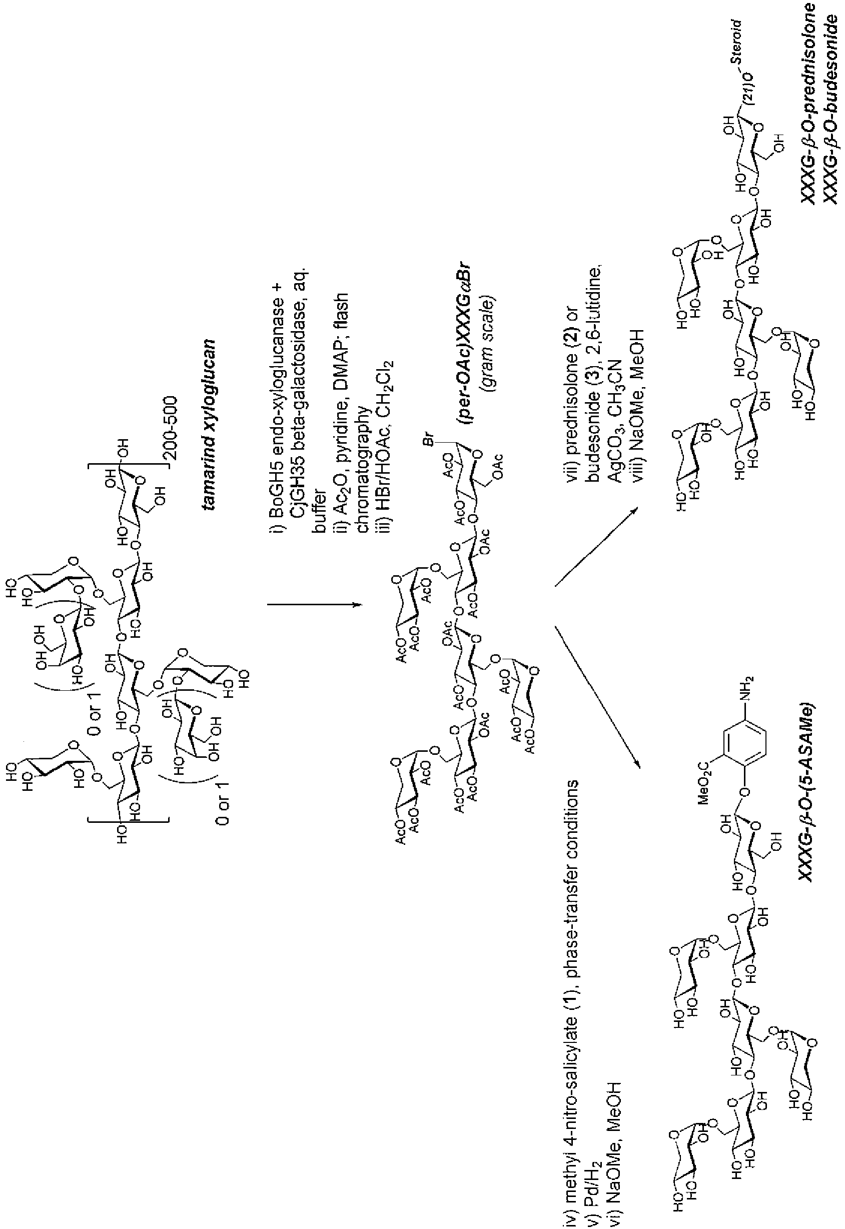

In step i) the β-galactosidase may be omitted to yield a mixture of variably galactosylated oligosaccharides, which may be fractionated, for examle by flash chromatography, following per-*O*-acetylation for further transformation. Note that the standard structural abbreviations used in the above synthetic scheme are G = D-Glc*p*; X = [α-D-Xyl*p*(1→6)]-Glc*p*(1→4); L = [*β*-D-Gal*p*(1-2)-*α*-D-Xyl*p*(1→6)]-Glc*p*(1→4).

As shown in the above synthetic scheme the protecting step can comprise acetylation of the hydroxyl groups of the xyloglucan, although other protecting groups/methods can be employed.

In accordance with certain embodiments, the glycosylation reaction of step (b) proceeds according to option iii) shown in the above synthetic scheme. For example, the glycosylation reaction can comprise: reacting the protected xyloglucan to form a glycosyl halide; and performing a substitution reaction with an alcohol-containing drug or drug derivative and the glycosyl halide. The glycosyl halide can be, for example, a glycosyl bromide.

In accordance with another embodiment, the glycosylation reaction comprises: reacting the protected xyloglucan to form a glycosyl halide; treating the glycosyl halide with Ag₂CO₃ to form a glycosyl alcohol; treating the glycosyl alcohol with trichloroacetonitrile to form an *O*-glycosyl trichloroacetimidate; and performing a substitution reaction with an alcohol-containing drug or drug derivative and the *O*-glycosyl trichloroacetimidate.

### 2. Conjugation to Xyloglucan via a Self-Immolative Linker

As described above, depending on the structure of the drug moiety, it can be useful to incorporate a self-immolative linker in the glyco-caged prodrug in order to facilitate conjugation to the drug moiety. This can be useful, for example, when the conjugation is via hydroxyl or amino groups of the drug moiety.

Self-immolative linker technology has been employed in the past to attach other chemical moieties. Examples of self-immolative linkers that can be successfully incorporated in the present glyco-caged prodrugs include, but are not limited to, carbamate linkers, carbamoyl linkers, and benzyl ester linkers.

One aspect provides a method for producing a prodrug compound of formula (II)

xyloglucan - *β* - O - L^{SI} - R^{D} (II)

wherein *β* - O represents a *β*-glycosidic bond, R^{D} is a drug moiety and L^{SI} is a self-immolative linker moiety, or a pharmaceutically acceptable salt or solvate thereof, which comprises: protecting the hydroxyl groups of a xyloglucan to form a protected xyloglucan; reacting the protected xyloglucan to add an appropriate leaving group moiety; performing a glycosylation reaction to form a glycosidic bond between the protected xyloglucan and a linker molecule to form a compound of formula (III)

xyloglucan - *β* - O - L^{SI} (III)

and attaching a drug or a drug derivative to L^{SI} of the compound of formula (III). Optionally, the step of adding the appropriate leaving group comprises first reacting the protected xyloglucan to form a glycosyl halide. The next step then comprises performing a substitution reaction with a linker molecule and the glycosyl halide.

The xyloglucan can be an XXXG, XXLG, XLXG, or XLLG-type xyloglucan, or it can be a mixture of two or more of XXXG, XXLG, XLXG, or XLLG-type xyloglucans. In certain embodiments, the xyloglucan is an XXXG-type.

The scheme provided below summarizes a synthetic method for producing phenyl glycoside/carbamate self-immolative linkers. In this synthetic scheme the drug moiety is represented by R or peptide.

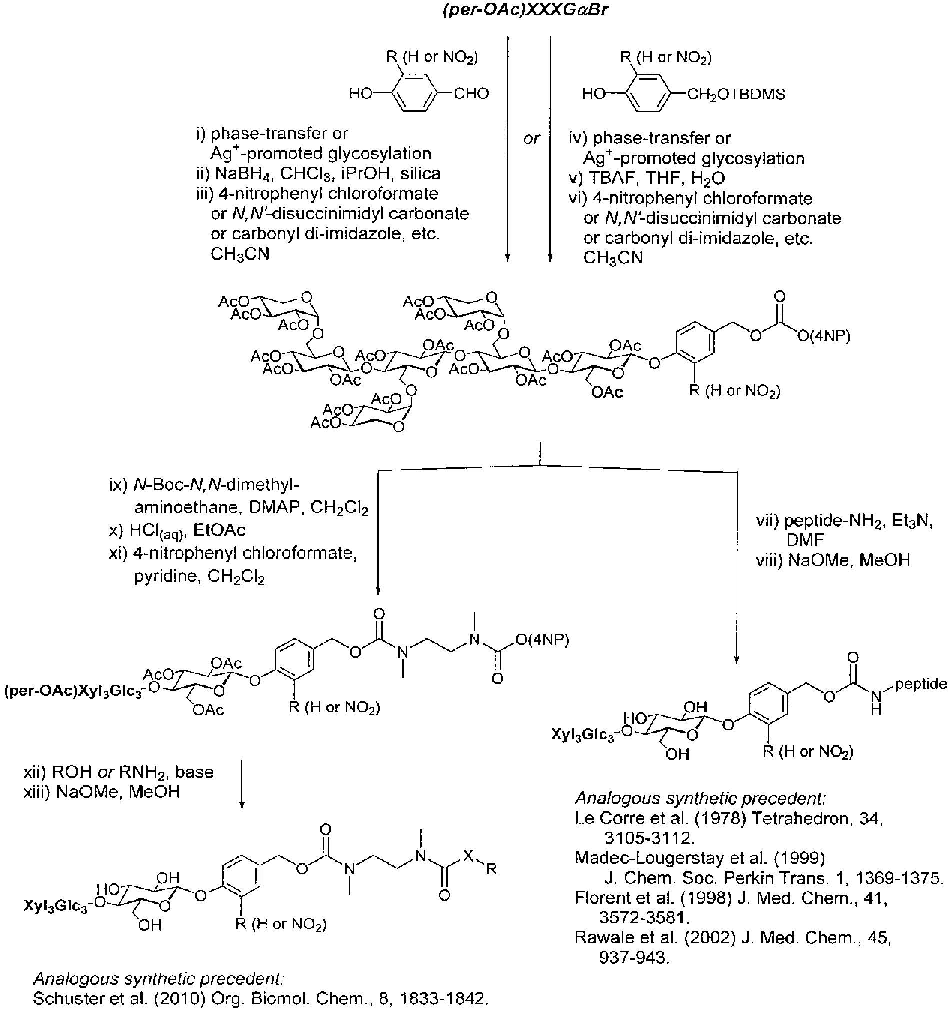

The scheme provided below summarizes a synthetic method for producing carbamoyl glycoside self-immolative linkers. In this synthetic scheme the drug moiety is represented by R.

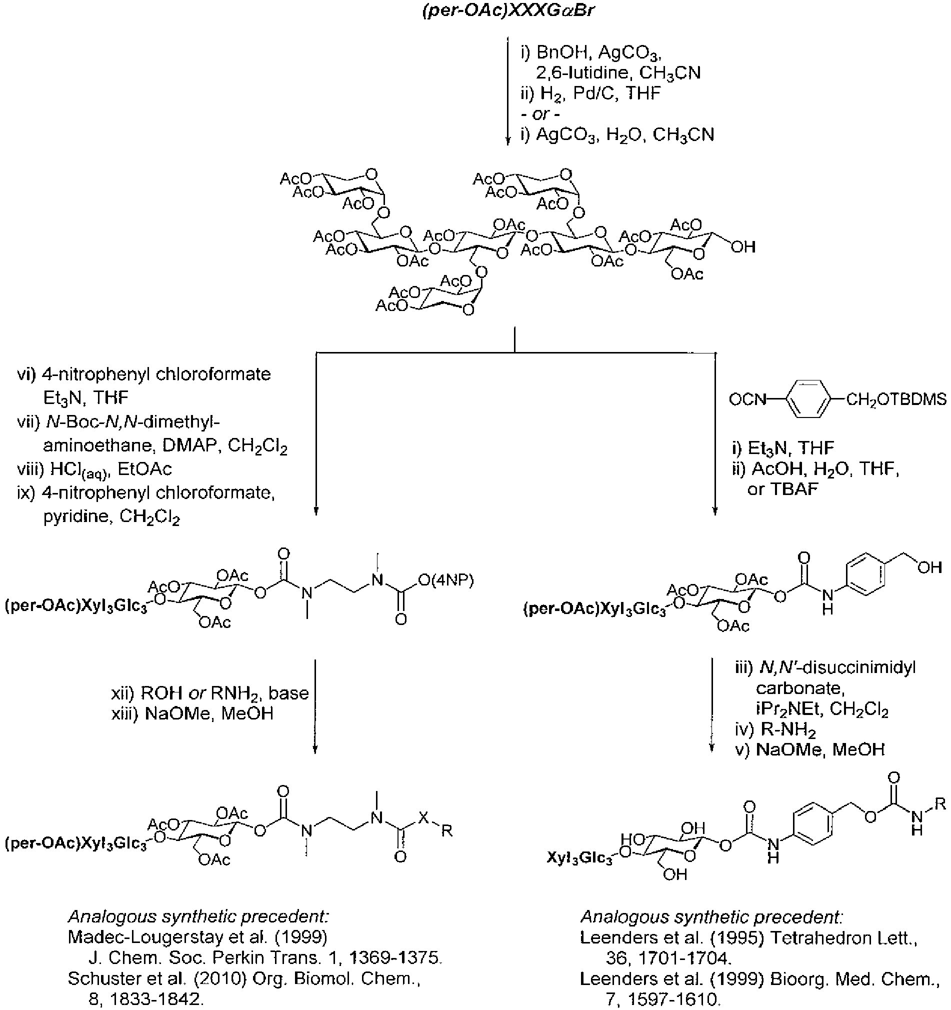

The scheme provided below summarizes a synthetic method for producing alkyl glycoside carbamate self-immolative linkers. In this synthetic scheme the drug moiety is represented by R or Drug.

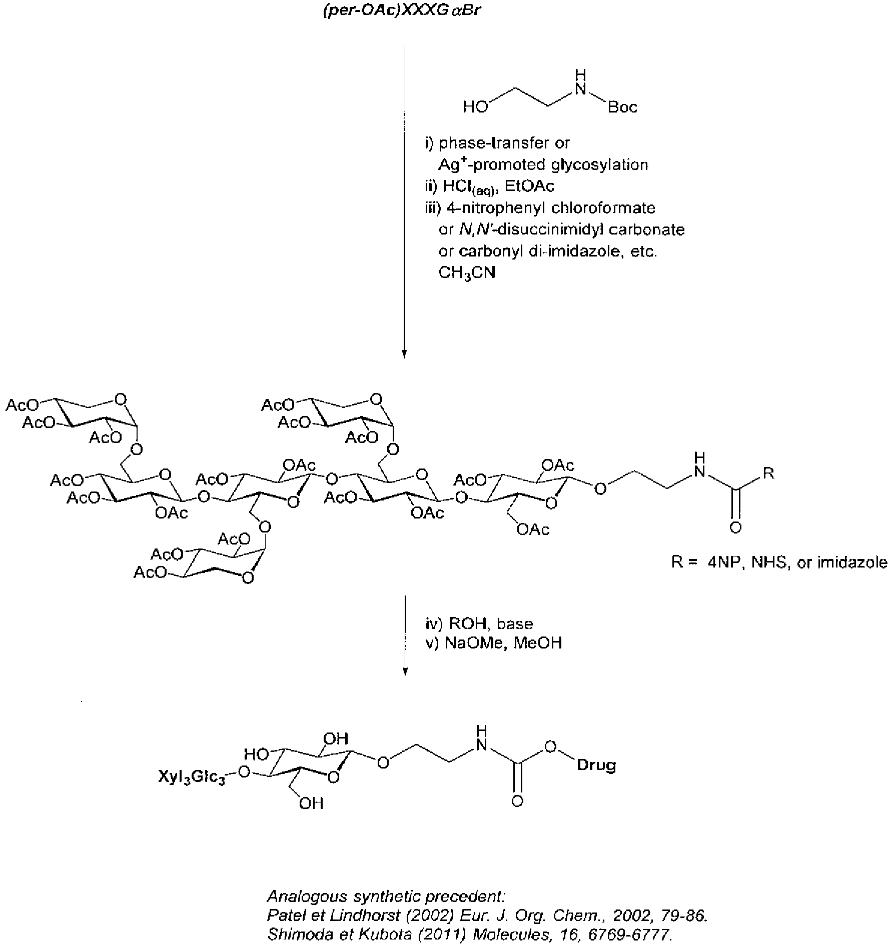

The scheme provided below summarizes a synthetic method for producing phenyl glycoside-benzyl ester self-immolative linkers. In this synthetic scheme the drug moiety is represented by NSAID as an example, however, other drug types can be incorporated in the glyco-caged prodrug produced using this synthetic method.

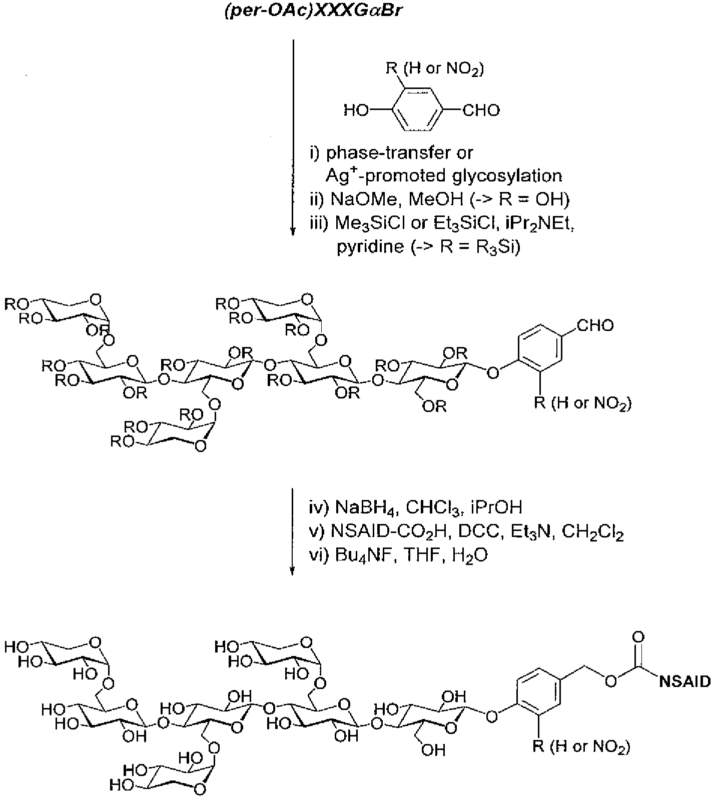

Although the above synthetic schemes indicate that the starting material is the per-acetylated XXXG bromide, it should be readily appreciated that other protected xyloglucan derivatives (i.e., in this case, xyloglucans derivatized with an appropriate leaving group), such as protected xyloglucan halides, can be readily employed as starting materials for these processes. Furthermore, the above synthetic schemes are merely illustrative of the methods employed to synthesize the present glyco-caged prodrugs and are not intended to limit the methods for manufacturing these prodrugs.

### Pharmaceutical Compositions

In another aspect, the present application provides a pharmaceutical composition comprising a glyco-caged prodrug, optionally in combination with a pharmaceutically acceptable carrier, diluent, excipient or medium, wherein the glyco-caged prodrug is a compound of formula (I) or formula (II):

xyloglucan - *β* - O - R^{D} (I)

xyloglucan - *β* - O - L^{SI} - R^{D} (II).

wherein *β* - O represents a *β*-glycosidic bond, R^{D} is a drug moiety and L^{SI} is a self-immolative linker, or a pharmaceutically acceptable salt or solvate thereof, wherein cleavage of the *β*-glycosidic bond connecting the xyloglucan to R^{D} or L^{SI} results in formation and release of a drug from the drug moiety

In certain embodiments, the pharmaceutical composition comprises a combination of two or more glyco-caged prodrugs.

In certain embodiments, the pharmaceutical composition comprises one or more of XXXG-*β*-O-dexamethasone (XXXG-DEXA), XXXG-*β*-O-prednisolone (XXXG-PRED), XXXG-*β*-O-budesonide (BUDE), XXXG-*β*-O-mesalazineME (XXXG-5ASAME), XXXG-*β*-O-mesalazine (XXXG-5ASA), or XXXG-SIL-*β*-O-mesalazine (XXXG-SIL-SASA).

The amount of glyco-caged prodrug in the pharmaceutical composition can be based on the amount of pharmaceutically active drug to be administered at the targetted lower gastrointestinal tract. In some embodiments, the amount of glyco-caged prodrug in the composition is based on an amount of the pharmaceutically active drug that is administered via conventional means since, in certain embodiments, the use of the glyco-cage can reduce the amount of active drug required to have the same pharmaceutical effect. As an example, the dosage range of both the active drug and prodrug forms of dexamethasone currently being investigated in mice is 0.05 - 2.0 mg/kg, and the range for prednisolone is 0.5 - 2.2 mg/kg. Dosages used in mice can be extrapolated to ranges for use in humans using standard dose conversion, of example based on FDA guidelines, or allometric scaling (see, for example, Nair A.B. and Jacob S. (2016) "A simple practice guide for dose conversion between animals and human." J. Basic Clin Pharma., Vol. 7: 27 - 31).

Suitable pharmaceutical compositions of the present application will generally include an amount of the glyco-caged prodrug(s) with an acceptable pharmaceutical diluent or excipient, such as a sterile aqueous solution, to give a range of final concentrations, depending on the intended use. The techniques of preparation are generally well known in the art, as exemplified by Remington's Pharmaceutical Sciences (18th Edition, Mack Publishing Company, 1995).

The compositions described herein may be administered systemically or locally, e.g.: orally (e.g., using capsules, powders, solutions, suspensions, tablets, sublingual tablets and the like). Preferably, the compositions are administered orally. In compositions for non-oral administration the glyco-caged prodrug can be formulated with one or more additives to improve uptake and/or bioavailabilty (e.g., DMSO, liposome, lipid, detergent, etc.).

In certain embodiments, the pharmaceutical compositions of the present application comprise a glyco-caged prodrug as the sole active ingredient and one or more other desired pharmaceutically inactive additives, excipients, and/or components (e.g., polymers, sterically hindered primary amines, cations, fillers, binders, carriers, excipients, diluents, disintegrating additives, lubricants, solvents, dispersants, coating additives, absorption promoting additives, controlled release additives, anti-caking additives, anti-microbial additives, preservatives, sweetening additives, colorants, flavors, desiccants, plasticizers, dyes, or the like), and no other active pharmaceutical ingredient(s). In other embodiments, the pharmaceutical composition comprises a combination of two or glyco-caged prodrugs, in combination with one or more other desired pharmaceutically inactive additives, excipients, and/or components.

The glyco-caged prodrug described herein can be combined with any pharmaceutically tolerable carrier or medium, e.g., solvents, dispersants, coatings, absorption promoting agents, controlled release agents, and one or more inert excipients (which include starches, polyols, granulating agents, microcrystalline cellulose, diluents, lubricants, binders, disintegrating agents, and the like), etc.

For treatment of gastrointestinal disorders, the glyco-caged prodrugs described herein are preferably administered orally, e.g., as a tablet, capsule, sachet containing a predetermined amount of the active ingredient pellet, gel, paste, syrup, bolus, electuary, slurry, powder, lyophilized powder, granules, as a solution or a suspension in an aqueous liquid or a non-aqueous liquid; as an oil-in-water liquid emulsion or a water-in-oil liquid emulsion, via a liposomal formulation or in some other form. Orally administered compositions can include binders, lubricants, inert diluents, lubricating, surface active or dispersing agents, flavoring agents, and humectants. Orally administered formulations such as tablets may optionally be coated or scored and may be formulated so as to provide sustained, delayed or controlled release of the active ingredient therein.

The glyco-caged prodrug can be co-administered with other agents used to treat gastrointestinal disorders including but not limited to the agents described herein.

In another aspect, suitable pharmaceutical compositions can comprise one or more other therapeutic agents. Such therapeutic agents include, without limitation, analgesic agents; anti-secretory agents; bile acid sequestrants; prokinetic and promotility agents; antibiotics; antiemetics; and mucosal-protecting agents.

As described above, the release of the pharmaceutically active drug from the glyco-caged prodrugs described herein requires the presence of a bacterial endo-xyloglucanase. In most instances, the bacterial endo-xyloglucanase is present in the lower gastrointestinal tract from the microflora that is naturally present in the subject (e.g., a mammallian or avian subject). In particular, the bacterial endo-xyloglucanase can be found in *Bacteroidetes,* such as *Bacteroides ovatus* or *Bacteroides uniformis,* or other *Bacteroides* species having a Xyloglucan Utilization Locus that is homologous to that found in *Bacteroides ovatus,* which are found in the native flora of the mammalian lower gastrointestinal tract, including in the swine or human lower gastrointestinal tract, or avian gastrointestinal tract (e.g., chicken).

By way of example, the glyco-caged prodrugs can be specifically hydrolysed by endo-xyloglucanases found in *Bacteroides ovatus, Bacteroides uniformis, Bacteroides cellulosyliticus, Bacteroides faecis*, *Bacteroides xylanisolvens, Bacteroides fluxus, Bacteroides coprocola* and *Bacteroides salanitronis,* which are found in the native flora of the subject's gastrointestinal tract, including in the mammalian (e.g., human) lower gastrointestinal tract. Other bacteria having an endo-xyloglucanase that can effectively hydrolyse xyloglucans, and therefore, the glyco-caged prodrugs of the present application can include, but are not limited to, *Bacillus parlicheniformis, Paeniclostridium sordellii, Parabacteroides distansonis, Prevotella* sp., *Salmonella enterica, Streptococcus gallolyticus* (e.g., subsp. *gallolyticus*).

In some subjects, such as those that have been treated, or are undergoing treatment, with antibiotics, the microflora is reduced such that there is insufficient bacterial endo-xyloglucanase present to effect the release of the pharmaceutically active drug from the glyco-caged prodrugs described herein. Furthermore, even in subjects that have a normal microflora, the release of the pharmaceutically active drug from the glyco-caged prodrugs can be increased or improved with increased amounts of bacterial endo-xyloglucanase in the lower gastrointestinal tract. Accordingly, in some embodiments, the pharmaceutical composition additionally comprises a probiotic to provide additional endo-xyloglucanase. In certain embodiments, the probiotic comprises a *Bacteroidetes* culture, such as a culture of *Bacteriodes,* for example *B. ovatus* or *B. uniformis,* or a combination thereof. In other embodiments, the probiotic comprises a culture of one of more bacteria that is a *Bacteroides* species having a Xyloglucan Utilization Locus that is homologous to that found in *Bacteroides ovatus.* In other embodiments, the probiotic comprises a culture of one or more bacteria selected from *Bacteroides ovatus, Bacteroides uniformis, Bacteroides cellulosyliticus, Bacteroides faecis*, *Bacteroides xylanisolvens, Bacteroides fluxus, Bacteroides coprocola, Bacteroides salanitronis, Bacillus parlicheniformis, Paeniclostridium sordellii, Parabacteroides distansonis, Prevotella* sp., *Salmonella enterica, Streptococcus gallolyticus* (e.g., subsp. *gallolyticus*).

Alternatively, the pharmaceutical composition is formulated for administration together with a probiotic. Again, the probiotic can comprise a *Bacteroidetes* culture, such as a culture of *B. ovatus* or *B. uniformis,* or a combination thereof. In other embodiments, the probiotic comprises a culture of one of more bacteria that is a *Bacteroides* species having a Xyloglucan Utilization Locus that is homologous to that found in *Bacteroides ovatus.* In other embodiments, the probiotic comprises a culture of one or more bacteria selected from *Bacteroides ovatus, Bacteroides uniformis, Bacteroides cellulosyliticus, Bacteroides faecis*, *Bacteroides xylanisolvens, Bacteroides fluxus, Bacteroides coprocola, Bacteroides salanitronis, Bacillus parlicheniformis*, *Paeniclostridium sordellii, Parabacteroides distansonis, Prevotella* sp., *Salmonella enterica, Streptococcus gallolyticus* (e.g., subsp. *gallolyticus*).

It should be readily understood that this the above teaching does not provide an exhaustive list of bacteria. Other bacteria that are not listed here, but that have endo-xyloglucanase activity, may be included in a probiotic as described herein.

### Method of Treatment

In various embodiments, the glyco-caged prodrugs described herein are useful for the prevention, reduction, alleviation, or treatment of a gastrointestinal disease or disorder. Accordingly, one aspect comprises a glyco-caged prodrug as described herein for use in a method of preventing, reducing, alleviating, or treating a gastrointestinal disease or disorder by administering the glyco-caged prodrug to a subject in need thereof.

In some embodiments, the gastrointestinal disorder is an enteric disease, such as inflammatory bowel disease. In specific embodiments, the gastrointestinal disorder Crohn's disease or ulcerative colitis.

In some embodiments, the method of preventing, reducing, alleviating, or treating the gastrointestinal disease or disorder additionally comprises administering a probiotic before, during or after administration of the glyco-caged prodrug. In specific embodiments, the probiotic is administered to the subject prior to administration of the glyco-caged prodrug in order to colonize the lower gastrointestinal tract with an endo-xyloglucanase producing bacteria, which may be the same or different from the bacteria present in the subject's microflora. As detailed above, the probiotic is administered in order to increase the amount of bacterial endo-xyloglucanase present in the lower gastrointestinal tract of the subject. In certain embodiments, the probiotic comprises a *Bacteroidetes* culture, such as a culture of *Bacteroides ovatus* or *Bacteroides uniformis,* or a combination thereof. In other embodiments, the probiotic comprises a culture of one of more bacteria that is a *Bacteroides* species having a Xyloglucan Utilization Locus that is homologous to that found in *Bacteroides ovatus.* In other embodiments, the probiotic comprises a culture of one or more bacteria selected from *Bacteroides ovatus, Bacteroides uniformis, Bacteroides cellulosyliticus, Bacteroides faecis*, *Bacteroides xylanisolvens, Bacteroides fluxus, Bacteroides coprocola, Bacteroides salanitronis, Bacillus parlicheniformis, Paeniclostridium sordellii, Parabacteroides distansonis, Prevotella* sp., *Salmonella enterica, Streptococcus gallolyticus* (e.g., subsp. *gallolyticus*).

To gain a better understanding of the invention described herein, the following examples are set forth. It should be understood that these examples are for illustrative purposes only.

### EXAMPLES

### EXAMPLE 1: Synthesis of XXXG-caged prodrugs

### Materials

All chemicals were purchased from established vendors and manufacturers.

### Chemical-enzymatic synthesis of glyco-caged prodrugs

Five glyco-caged prodrugs, XXXG-*β*-O-dexamethasone (XXXG-DEXA), XXXG-*β*-O-prednisolone (XXXG-PRED), XXXG-*β*-O-budesonide (BUDE), XXXG-*β*-O-mesalazineME (XXXG-5ASAME), and XXXG-*β*-O-mesalazine (XXXG-5ASA), and (Table 1), have been synthesized via three conjugation methodologies - Koenigs-Knorr, TCA-imidate, and phase transfer. Their syntheses are described in Schemes 1, 2, 3, and 4, beginning from readily available powdered tamarind seed kernel powder. Where problems may arise due to functional group compatibilities to the reagents used, these functional groups were protected with an appropriate protecting group by a known method in advance. These protecting groups were subsequently removed by known methods. The reaction conditions were optimized to increase the combined overall yield and/or to minimize environmental impacts.

Table 1 provides a summary of the glyco-caged prodrugs synthesized in this study.

**Table 1: Glyco-caged prodrugs**

| **Compound** | **Abbreviation** | **Chemical Structure** |
|---|---|---|
| XXXG-*β*-O-mesalazineME | XXXG-5ASAME | 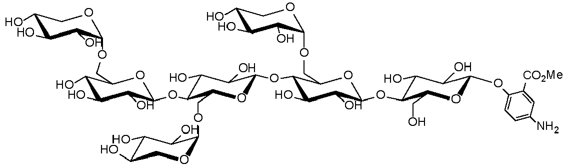 |
| XXXG-*β*-O-mesalazine | XXXG-SASA | 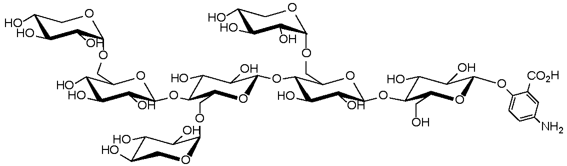 |
| XXXG-*β*-O-prednisolone | XXXG-PRED | 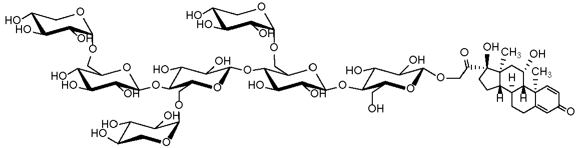 |
| XXXG-*β*-O-budesonide | XXXG-BUDE | 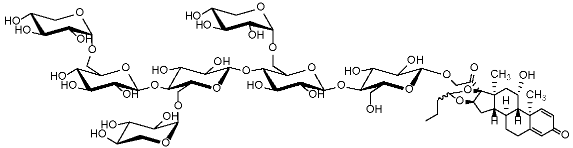 |
| XXXG-*β*-O-dexamethasone | XXXG-DEXA | 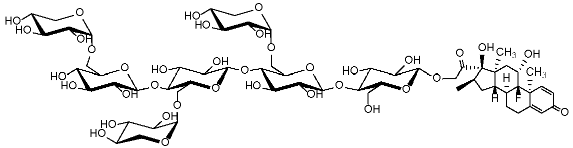 |

### Synthesis of XXXG

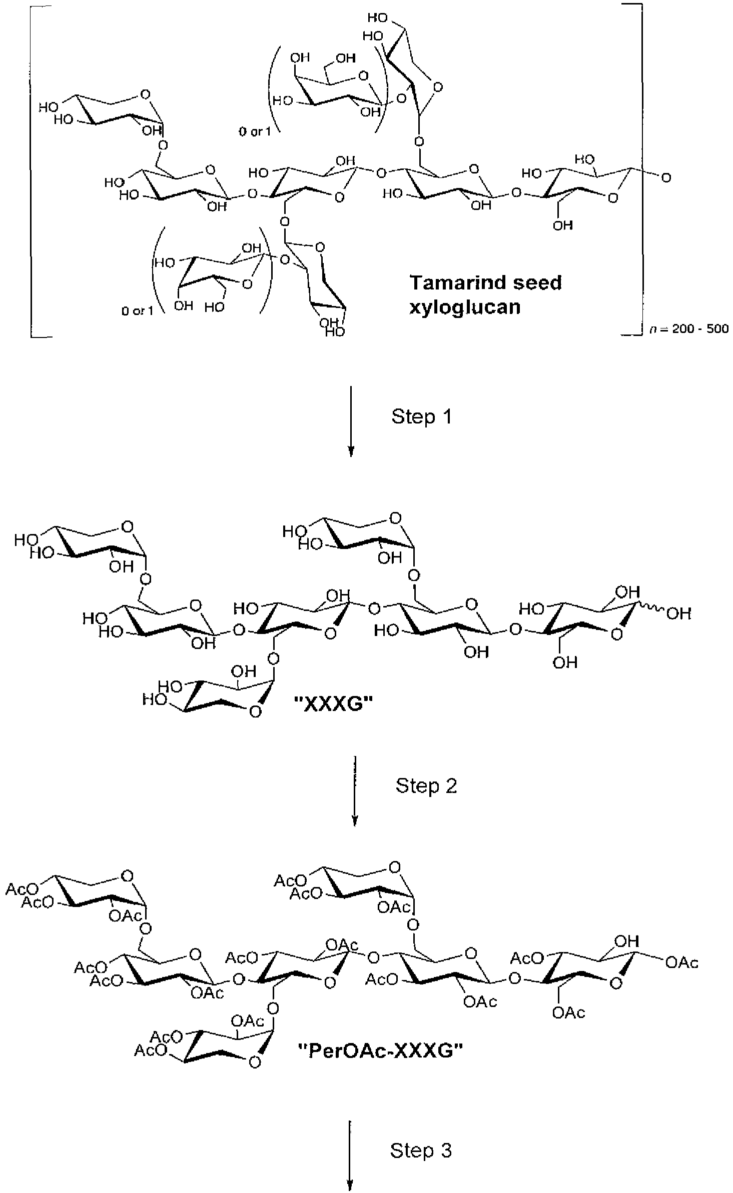

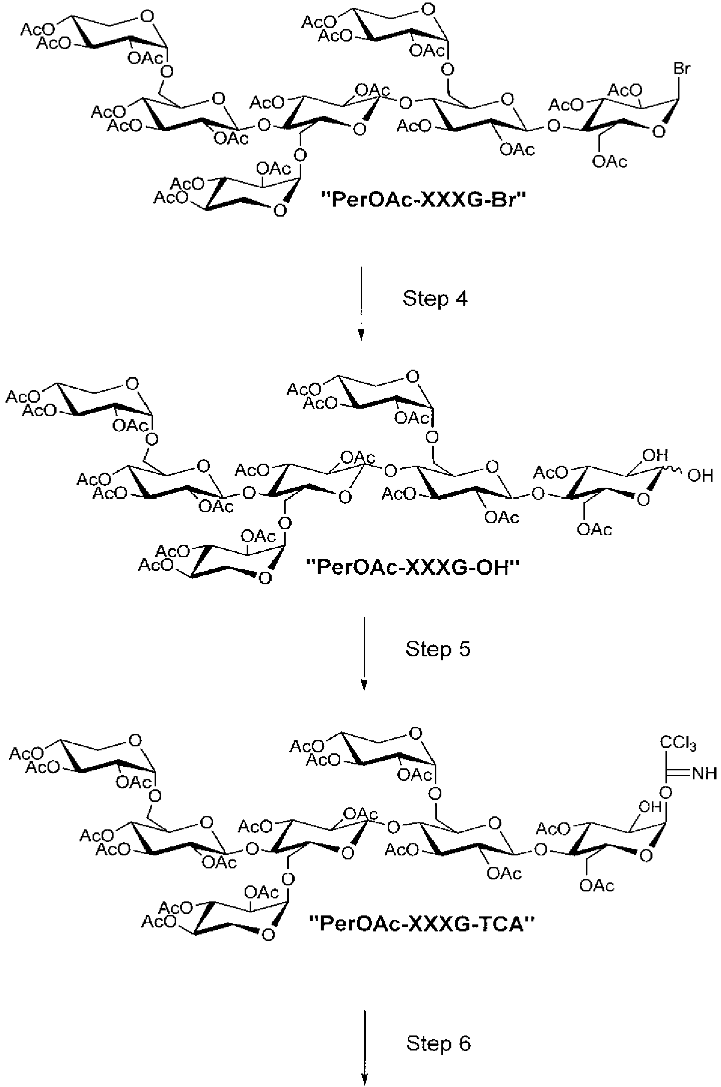

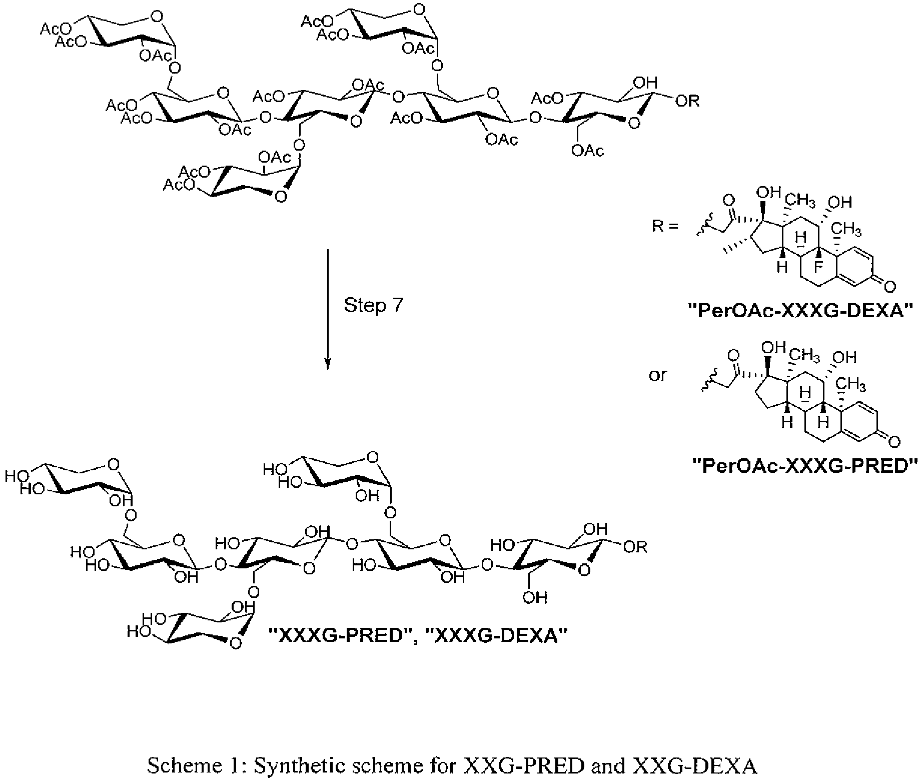

### Scheme 1. Step 1

This step is the enzymatic digestion of xyloglucan from powdered tamarind seed kernels by the action of cellulase from the fungus, *Trichoderma reesei.* This process produces a mixture of several xyloglucan oligosaccharides (XyGOs) that includes XXXG after an average time period of 6 hrs. The mixture is further treated with *β*-galactosidase enzyme from the fungus, *Aspergillus niger,* providing XXXG as the major product after about 12 - 24 hrs. Other enzymes that could be used in the hydrolysis include but are not limited to various broad specificity endo-glucanases/cellulases or specific endo-xyloglucanases (e.g., BoGH5 (Larsbrink J. et al. (2014) "A discrete genetic locus confers xyloglucan metabolism in select human gut Bacteroidetes." Nature, 506, 498-502), CjGH5D (Attia M.A. et al. (2018) "In vitro and in vivo characterization of three Cellvibrio japonicus Glycoside Hydrolase Family 5 members reveals potent xyloglucan backbone-cleaving functions." Biotechnol. Biofuels, 11, 45), PoGH74 (Arnal G. et al. (2018) "Structural enzymology reveals the molecular basis of substrate regiospecificity and processivity of an exemplar bacterial glycoside hydrolase family 74 endo-xyloglucanase." Biochem. J., 475, 3963-3978.), etc. (Attia, M.A. and Brumer, H. (2016) "Recent structural insights into the enzymology of the ubiquitous plant cell wall glycan xyloglucan." Curr. Opin. Struct. Biol., 40, 43-53; and Eklöf, J.M., Ruda, M., Brumer, H.* (2012) Distinguishing xyloglucanase activity in endo-beta(1-4)glucanases. Method. Enzymol., Vol. 510, Ch. 6, pp. 97-120)) and various beta-galactosidases (e.g. CjBgl35A (Larsbrink J. et al. (2014) "A complex gene locus enables xyloglucan utilization in the model saprophyte Cellvibrio japonicus. Mol. Microbiol., 94, 418-433"), BoGH2 (Larsbrink J. et al. (2014) "A discrete genetic locus confers xyloglucan metabolism in select human gut Bacteroidetes." Nature, 506, 498-502), etc. (Attia, M.A. and Brumer, H. (2016) "Recent structural insights into the enzymology of the ubiquitous plant cell wall glycan xyloglucan." Curr. Opin. Struct. Biol., 40, 43-53)). These reactions are typically performed in buffered conditions (pH = 5 to 6.5) and at temperatures of between 37 - 58°C in aqueous solution.

Procedure: De-oiled tamarind kernel powder (20 g) was suspended in 2 L purified Milli-Q water and stirred overnight at 60 °C. The suspension was centrifuged and the supernatant decanted into an autoclaved 2 L Erlenmeyer flask. 15 µL of 1 M ammonium acetate buffer and 33 mg of *T. reesei* cellulase was added, and the mixture was stirred at 45 °C. After 6 hrs, 0.31 mL of *Aspergillus niger β*-galactosidase was added and the mixture was stirred for a further 24 hrs at 45 °C. The mixture was then heated at 80 °C for 20 mins, and concentrated *in vacuo.* The crude material was then lyophilized to provide 13 g of crude XXXG, confirmed by HPLC analysis.

### Synthesis of Per-O-Ac-XXXG

### Scheme 1, Step 2

This step is the acetylation of all hydroxyl groups present in XXXG (per-*O-*acetylation) and can be carried out either under acidic or basic conditions with the corresponding catalyst. The most common acetylating agent is acetic anhydride, although more reactive agents e.g. acetyl chloride are sometimes used for more difficult substrates but may lead to side products. The base catalyst is usually 4-DMAP (4-dimethylaminopyridine) and loads typically range from 1 - 5 mol %. The reaction solvent is usually pyridine although the use of other solvents is not uncommon as long as they are not involved in the reaction. Heating is required in the reaction, and temperatures range from 40 - 60 °C. Reaction times are variable and dependent on the starting material, but range from 3-24 hrs.

Procedure A: 5 g of crude XXXG obtained in Step 1 was suspended in 30 mL of DMF and heated with stirring at 80 °C for 15 to 20 mins. The suspension was centrifuged for 20 mins at 4000 g and decanted into a heat-dried round-bottom flask. 100 mL of acetic anhydride, 100 mL of pyridine and 50 mg of 4-DMAP were then added, and the mixture was stirred at 60 °C for 16 hrs, whereupon it was concentrated *in vacuo* and the crude product triturated with 50 mL dichloromethane. This solution was then washed with 3 × 100 mL of water, 100 mL of 5% lithium chloride solution and 100 mL of saturated sodium bicarbonate solution. The washed solution was then dried over anhydrous magnesium sulfate and concentrated *in vacuo* to provide 3 g of crude material, which was purified with flash column silica gel chromatography using 2:1 toluene: acetone as the eluent to afford 2.4 g of pure Per-*O*-Ac-XXXG.

Procedure B: 6.0 g of crude XXXG obtained in Step 1 was suspended in 50 mL of AcOH. To this mixture 40 mL of acetic anhydride was added. The stirring was continued for one hr before catalytic amount of sulfuric acid was added dropwise. The progress of the acetylation was monitored via thin layer chromatography. After 4-6 hrs, the acetylation was complete and the reaction was quenched with 1800 mL of water/ice. A heavy white precipitate appeared at this point, and the stirring was continued till all the ice had been melted. The white suspension was filtered. The filter cake was washed with deionized water (30 mL × 3) and air dried in a fume hood overnight. Crude Per-*O*-Ac-XXXG thus obtained (9.86 g) was loaded to a silica gel column. Gradient elution with toluene/acetone (5:1 to 2:1) afforded 8.6 g of pure Per-*O*-Ac-XXXG as evidenced by thin layer chromatography and MALAI mass spectrometry. TLC single spot R*_{f}* = 0.25 (toluene/acetone = 2:1); m/z (MALDI) 1925.5 (M + Na⁺, C₇₉ H₁₀₆O₅₃Na requires 1925.5).

### Synthesis of Per-O-Ac-XXXG-Br

### Scheme 1, Step 3

This step is the anomeric bromination of Per-*O*-Ac-XXXG by treatment with a solution of hydrogen bromide in acetic acid; a well-known method to access α-anomers. The reaction is usually carried out in the cold (0 °C) and with a large excess of hydrogen bromide, typically 20 - 30 molar equivalents to ensure complete bromination within reaction times of between 1 - 3 hrs. Solvents used are typically chlorinated with dichloromethane the most common although others such as chloroform and 1,2-dichloroethane are also used.

Procedure A: To a solution of Per-*O*-Ac-XXXG (0.5 g, 0.28 mmol) obtained in Step 2 in dichloromethane at 0 °C was added to 2.5 mL of 33 wt % hydrogen bromide in acetic acid, and the mixture was stirred for 2 hrs, whereupon it was poured into 20 g of ice and extracted with 2 × 30 mL of dichloromethane. The combined dichloromethane extract was then washed with 2 x 40 mL of cold water, 2 x 40 mL cold saturated sodium bicarbonate and dried over anhydrous magnesium sulfate. The extract was then concentrated *in vacuo* to provide 0.48 g of crude material, which was purified with flash column silica gel column chromatography using step gradient elution with 3:1 toluene: acetone to 2:1 toluene acetone to afford 0.35 g of Per-*O*-Ac-XXXG-Br.

Procedure B: To a solution of Per-*O*-Ac-XXXG (0.21 g, 0.11 mmol) obtained in Step 2 in 5 mL of dichloromethane/AcOEt (10:1) at room temperature was added 367 mg ( 1 mmol) of TiBr₄ and argon. The mixture was stirred in the dark for 68 hrs, whereupon it was poured into 100 g of ice/water and extracted with 2 × 30 mL of dichloromethane. The combined dichloromethane extract was then washed with 2 x 40 mL cold water, 2 x 40 mL cold saturated sodium bicarbonate and dried over anhydrous magnesium sulfate. The extract was then concentrated *in vacuo* to provide 0.22 g of crude material, which was purified with flash column silica gel column chromatography using step gradient elution with 3:1 toluene: acetone to 2:1 toluene acetone to afford 0.18 g of pure Per-*O*-Ac-XXXG-Br as evidenced by TLC and mass spectrometry. TLC single spot R_{f} = 0.26 (toluene/acetone = 2:1); m/z (MALDI) 1945.3 (M + Na⁺, C₇₉ H₁₀₆O₅₃Na requires 1945.4).

### Synthesis of Per-O-Ac-XXXG-OH

### Scheme 1, Step 4

The freshly made Per-*O*-Ac-XXXG-Br (1.3 g, 676 mg) was dissolved in 25 mL of acetone, followed by the addition of 5 mL of water. To this mixture was added 0.77 g of AgCO₃, and the resultant suspension was stirred in dark overnight. The reaction progress was monitored via thin layer chromatography until the TLC test indicated the selective hydrolysis was complete. The suspension was then filtered and the filtrate was concentrated under reduced pressure. The crude thus obtained was loaded onto a silica gel column. Eluting the column with toluene/acetone (4:1 to 2:1) afforded 1.18 g of pure Per-*O*-Ac-XXXG-OH.

### Synthesis of Per-O-Ac-XXXG-TCA

### Scheme 1, Step 5

Under a stream of argon, TCA (trichloroacetonitrile, 0.45 mL, 4.49 mmol) and DBU (1,8-diazabicyclo[5,4,0]-undec-7-ene, 10 µl) were added to a solution of Per-*O*-Ac-XXXG-OH (0.39 g, 0.20 mmol) in anhydrous dichloromethane (DCM), and the reaction was stirred for 2 hrs at -30°C until a TLC test indicated the complete consumption of the starting material. The volatiles were removed under reduced pressure, and the crude thus obtained was purified via silica gel flash chromatography, which afforded 0.3 g of Per-*O*-Ac-XXXG-TCA.

### Synthesis of Per-O-Ac- XXXG-β-O-dexamethasone

### Scheme 1, Step 6

Per-*O*-Ac-XXXG-TCA (307 mg, 0.15 mmol) was dissolved in 12 mL of anhydrous DCM, followed by the addition of 3Å MS (molecular sieve). The suspension was stirred at room temperature for 0.5 hr before it was cooled down to -30 °C. BF₃·Et₂O (50 µl) was added to this mixture dropwise under argon. The reaction was allowed to proceed for 4-20 hrs until a TLC test indicated the complete consumption of the starting material. Triethylamine (1 mL) was added to the reaction mixture at 30 °C to quench the reaction. DCM (50 mL) was added and the reaction mixture was allowed to warm to room temperature. The suspension was then filtered, washed with DCM (20 mL X 3). The filtrate was concentrated to afford 480 mg of crude as a white solid which was purified via silica gel flash chromatography (toluene/acetone 4:1 to 2:1). The resultant Per-*O*-Ac-XXXG-β-O-dexamethasone (140 mg) was characterized by thin layer chromatography, proton NMR and MALDI mass spectrometry. Rf = 0.26 (toluene/acetone 2:1); m/z 2257.6 (M + Na⁺, C₉₉H₁₃₁FO₅₆Na requires 2257.7).

### Synthesis of XXXG-β-O-dexamethasone

### Scheme 1, Step 7

Per-O-Ac-XXXG-β-O-dexamethasone (140 mg, 0.062 mmol) was dissolved in a mixture of methanol and dichloromethane (6 mL, 4:1) at room temperature under stirring. To this mixture 15 µL (0.07 mmol) of a 25%(w/w) solution of sodium methoxide in methanol was added. The reaction was allowed to continue for 17 hrs until a TLC test indicated the complete consumption of the starting material. 200 mg of Amberlite^{™} IR-120 resin (H⁺ form) was added and stirred for 10 min, whereupon the suspension was filtered and the filtrate was concentrated under reduced pressure. The crude material was then purified with flash column silica gel chromatography using ethyl acetate: isopropanol: water (3:2:1) as the eluent to afford 65 mg (0.045 mmol) of pure XXXG-β-O-dexamethasone. m/z 1459.4 (M + Na⁺, C₆₁H₉₃FO₃₇Na requires 1459.5).

### Synthesis of XXXG-β-O-prednisolone

The XXXG-β-O-prednisolone was obtained in a manner similar to that described above for the synthesis of XXXG-β-O-dexamethasone. 200 mg of XXXG-β-O-prednisolone was obtained by following the general procedure described in scheme 1, step 1-7.

### Synthesis of XXXG-β-O-prednisolone and XXXG-β-O-budesonide

The XXXG-β-O-prednisolone was obtained in a manner similar to that described above for the synthesis of XXXG-β-O-dexamethasone.

### Alternative Synthesis of XXXG-β-O-prednisolone and XXXG-β-O-budesonide

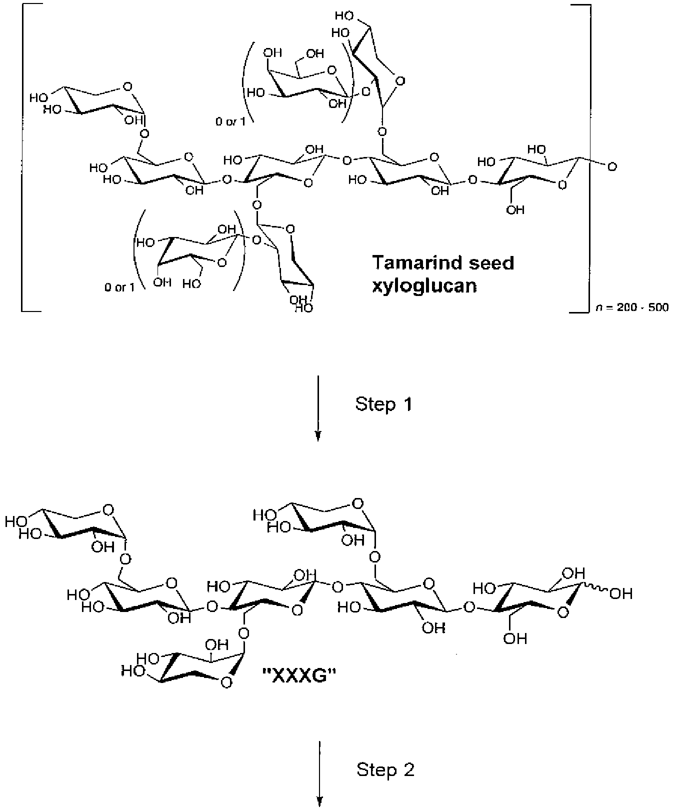

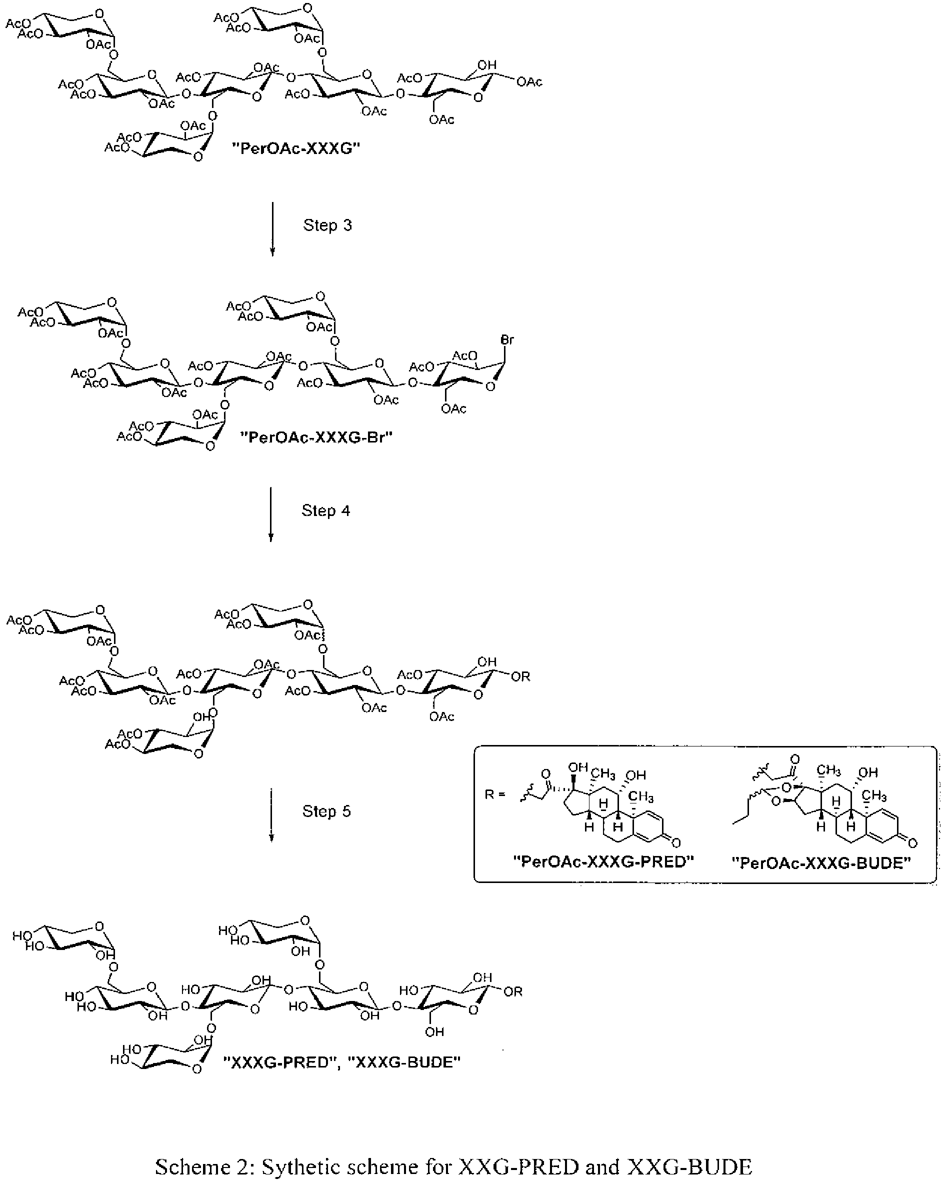

Steps 1 - 3 of scheme 2 were performed in the same manner as described above in relation to Scheme 1.

### Scheme 2, Step 4

This step is the glycosylation of the known corticosteroidal IBD drugs prednisolone and budesonide with Per-*O*-Ac-XXXG-Br prepared in Step 3 via the classical Koenigs-Knorr reaction. The reaction involves activation of the anomeric position by a suitable promoter reagent, typically silver (1) salts such as silver (I) carbonate, silver (I) oxide and silver (I) trifluoromethanesulfonate, to generate a reactive oxocarbenium intermediate. If neighbouring group participation is present, a stereoselective glycosylation usually occurred to afford the *β*-glycoside. Typical loads for the promoter are between 1 - 3 molar equivalents with respect to the glycosyl donor. It is advantageous to introduce an excess (1.5 - 3 molar equivalents) of the glycosyl acceptor to drive consumption of the donor to completion. Common solvents used in this reaction are chlorinated, and include, for example, dichloromethane, chloroform and carbon tetrachloride. Reaction temperatures can range between ambient and reflux. Reaction times vary with the starting material but are usually between 5 - 20 hrs.

Procedure (for preparation of Per-*O*-Ac-XXXG-PRED): To a solution of Per-*O*-Ac-XXXG-Br (170 mg, 0.09 mmol) obtained in Step 3 in anhydrous 1,2-dichloroethane (4 mL) was added prednisolone (48 mg, 0.13 mmol) and 4A molecular sieves, and the mixture was stirred at room temperature for 30 mins. Silver (I) trifluoromethanesulfonate (34 mg, 0.13 mmol) was then added and the mixture was stirred in the dark at 80 °C for 7 hrs, whereupon it was cooled to room temperature, diluted with dichloromethane and filtered through a Celite^{™} pad. This solution was washed with 2 × 40 mL saturated sodium bicarbonate and 40 mL of brine and dried over anhydrous magnesium sulfate, whereupon it was concentrated *in vacuo* to provide 158 mg of crude material, which was purified with flash column silica gel chromatography using step gradient elution with 99:1 - 98:2 - 97:3 dichloromethane:methanol to afford 78.6 mg of a mixture of Per-*O*-Ac-XXXG-PRED and Per-*O*-Ac-XXXG-OH, a byproduct arising from the hydrolysis of unreacted intermediate during work-up. It was not possible to separate these two species via column chromatography.

The preparation of Per-*O*-Ac-XXXG-BUDE was carried out by the same procedure as above, whereby 270.1 mg, 0.14 mmol of Per-*O*-Ac-XXXG-Br was treated with 90.8 mg, 0.21 mmol of budesonide in the presence of 54.2 mg, 0.21 mmol of silver (I) trifluoromethanesulfonate to provide 287 mg of crude material, which was then purified to give 145.9 mg of a mixture of Per-*O*-Ac-XXXG-BUDE and Per-*O*-Ac-XXXG-OH. This mixture was used in the de-acetylation without further purification.

### Scheme 2, Step 5

This step is the global deacetylation of the glycosides prepared in Step 4 by the action of sodium methoxide. Only a catalytic amount ranging from 20 - 50 mol % of sodium methoxide is required as it is continually regenerated from the bulk solvent (methanol). The reaction is usually carried out at ambient temperature and reaction times vary with starting materials, but are typically between 1 - 20 hrs.

Procedure (for preparation of XXXG-PRED): To the mixture of Per-*O*-Ac-XXXG-PRED and Per-*O*-Ac-XXXG-OH obtained in Step 4 (78.6 mg, 0.036 mmol calculated w.r.t. Per-*O*-Ac-XXXG-PRED) in 10% dichloromethane in methanol (2.4 mL) was added via micropipette 1.5 µL (0.007 mmol) of a 25 wt % solution of sodium methoxide in methanol. The mixture was stirred overnight for 17 hrs. 200 mg of Amberlite^{™} IR-120 H⁺ form resin was added and stirred for 10 min, whereupon the solvent was removed *in vacuo.* The crude material was then purified with flash column silica gel chromatography using 3:2:1 ethyl acetate:isopropanol:water as the eluant to afford 24.3 mg of pure XXXG-PRED.

The preparation of XXXG-BUDE was carried out by the same procedure as above, whereby the mixture of Per-*O*-Ac-XXXG-BUDE and Per-*O*-Ac-XXXG-OH obtained in Step 4 (145.9 mg, 0.064 mmol calculated w.r.t. PerOAc-XXXG-BUDE) was treated with 2.7 µl of 25 wt % solution of sodium methoxide in methanol. The crude material obtained on work-up was purified to afford 65.3 mg of pure XXXG-BUDE.

### Synthesis of XXXG-5ASAME

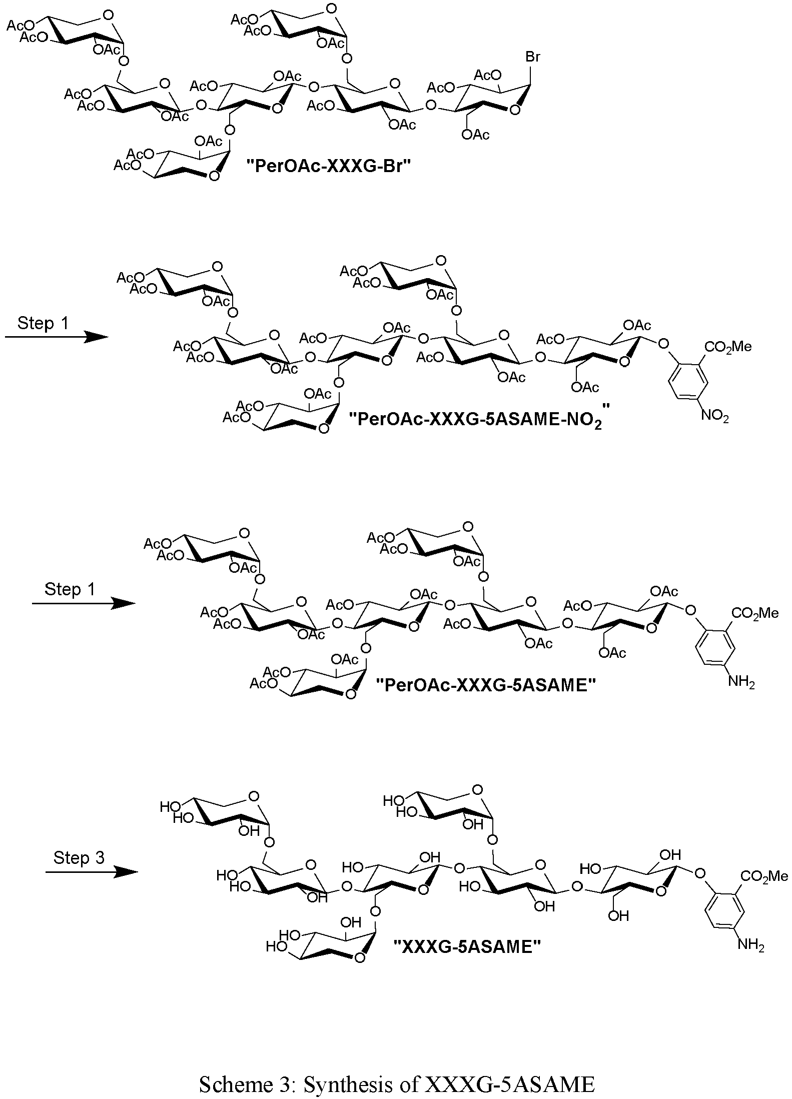

### Scheme 3. Step 1

This step is the glycosylation of the known IBD drug methyl 5-aminosalicylate. It is an alternative method to the Koenigs-Knorr reaction for the glycosylation of phenolates. In this reaction, the sodium salt of methyl 5-nitrosalicylate was coupled with Per-*O*-Ac-XXXG-Br obtained in Step 3 (Scheme 1). The reaction is carried out in a biphasic medium consisting of water and dichloromethane and reactant transfer is facilitated by the use of a phase transfer catalyst. Typical loads for the acceptor are between 1.5 - 3 molar equivalents with respect to the donor, and 0.5 - 1 molar equivalent with respect to the donor for the phase transfer catalyst. The reaction is usually carried out at ambient temperature and typical reaction times vary with starting material but is usually in the range of 24 - 48 hrs.

Procedure: To a solution of Per-*O*-Ac-XXXG-Br (160 mg, 0.085 mmol) and benzyltributylammonium chloride (27 mg, 0.085 mmol) in dichloromethane (2 mL) was added a solution of methyl 2-nitrosalicylate sodium salt (56 mg, 0.26 mmol) in 7 mL water. The mixture was stirred at room temperature for 48 hrs, whereupon it was diluted with water and dichloromethane. The dichloromethane layer was extracted with 2 x 20 mL water, washed with brine and dried over anhydrous magnesium sulfate. Concentration of the solvent *in vacuo* provided 190 mg of crude material, which was purified with flash column silica gel chromatography using step gradient elution with 3:1 - 2:1 toluene: acetone to afford 130 mg of Per-*O*-Ac-XXXG-5ASAME-NO₂.

### Scheme 3, Step 2

This step is the hydrogenation of Per-*O*-Ac-XXXG-5ASAME-NO₂ and involves the reduction of the nitro functional group therein. The reaction is performed in the presence of a metal catalyst in a suitable solvent, usually methanol, under a hydrogen atmosphere of 1 atm and at temperatures ranging from ambient to 100 °C. Typical catalyst loads fall between 1 - 10 wt % and reaction times vary with starting material and temperature, but are usually between 3 to 20 hrs. Examples of suitable catalysts include palladium on carbon, platinum on carbon and platinum dioxide.

Procedure: To a solution of Per-*O*-Ac-XXXG-5ASAME-NO₂ (124 mg, 0.061 mmol) obtained in Step a in anhydrous, degassed methanol (2.3 mL) was added 12.4 mg of 10% palladium on carbon, and the mixture was stirred at 40 °C for 3 hrs. The mixture was suction filtered through a Celite pad and the solvent was removed *in vacuo* to give 110 mg of crude material, which was subsequently purified with flash column silica gel chromatography (the silica gel was pre-neutralized with a 2% triethylamine in hexane solution) using 2:1 toluene:acetone as the eluant to afford 72 mg of Per-*O*-Ac-XXXG-5ASAME.

### Scheme 3, Step 3

This step involves the global deacetylation of Per-*O*-Ac-XXXG-5ASAME with catalytic sodium methoxide.

Procedure: To a solution of Per-*O*-Ac-XXXG-5ASAME obtained in Step 2 above (71.1 mg, 0.035 mmol) in 10% dichloromethane in methanol (2.4 mL) was added via micropipette 3.75 µL (0.0017 mmol) of a 25 wt % solution of sodium methoxide in methanol. The mixture was stirred for 3 hrs, whereupon 200 mg of Amberlite^{™} IR-120 H⁺ form resin was added and stirred for 10 min. The solvent was removed *in vacuo* and the residue redissolved in water. The solution was filtered and lyophilized to afford 34 mg of crude XXXG-5ASAME. The pure XXXG-5ASAME (30 mg) was obtained after subjecting the above crude to reverse phase chromatography.

### Synthesis of XXXG-SASA

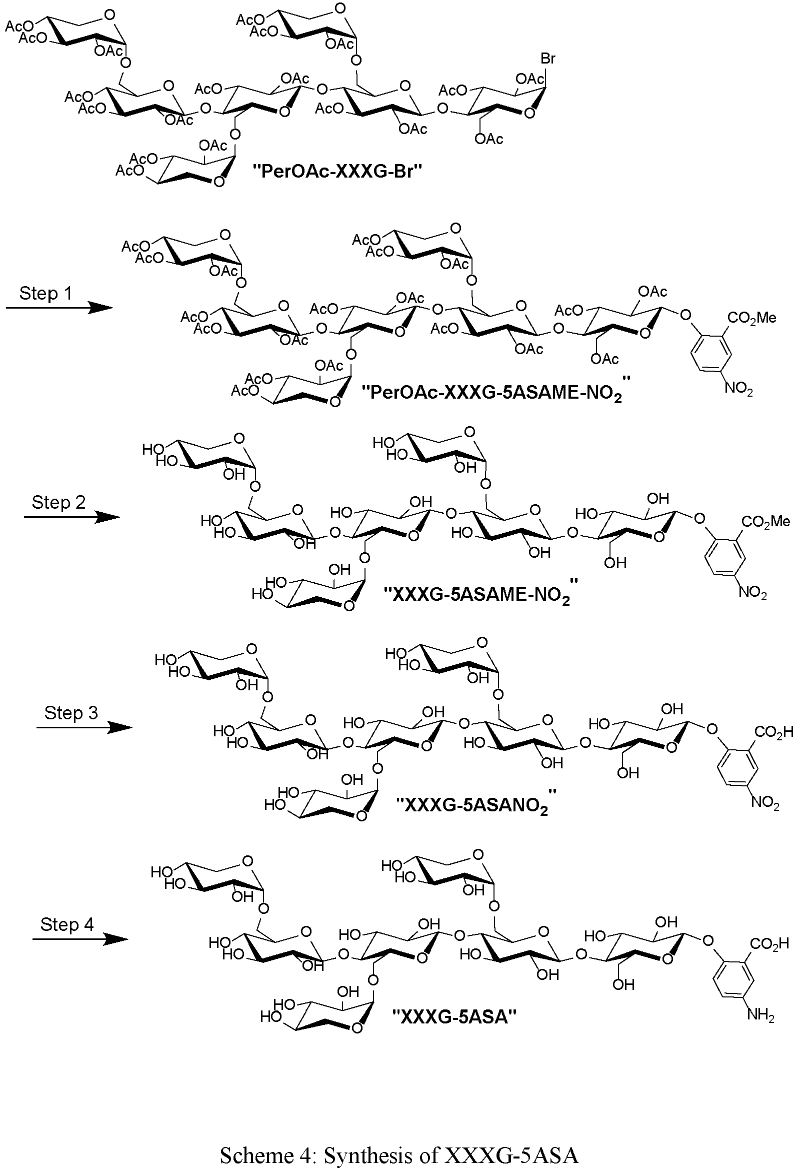

### Scheme 4, Step 1

To the freshly made Per-*O*-Ac-XXXG-Br (417 mg) were added 10 mL of dry acetonitrile, 67 mg of 5-ASAME-NO₂ and 1 g of 3Å MS. The suspension was stirred under argon for 0.5 h before the addition Ag₂O (100 mg) and 2,6 - lutidine (0.8 mL). The resultant suspension was stirred in the dark overnight at room temperature. 2 g of silica gel was added and the volatiles were removed under reduced pressure. The resultant solid was loaded onto the top of a prepared silica gel column. Elution with toluene/acetone (4:1 to 2:1) afforded 240 mg of Per-*O*-AcXXXG-5ASAME-NO₂. m/z 2062.6 (M + Na+, C₈₅H₁₀₉NO₅₆Na requires 2062.6).

### Scheme 4, Step 2

Per-*O*-Ac-XXXG-5ASAME-NO₂ (240 mg) was dissolved in a mixture of methanol and DCM (4:1, 6 mL). Catalytic amount of sodium methoxide (2 mg) was added and the resultant suspension was stirred overnight before all volatiles were removed under reduced pressure at 40°C. The crude (180 mg, XXXG-5ASAME-NO₂) thus obtained was used in the next step without further purification.

### Scheme 4, Step 3

The crude XXXG-5ASAME-NO₂ (180 mg) obtained in the previous step was dissolved in 1 N sodium hydroxide (8 mL) and the resultant solution was warmed to 45°C under stirring. The reaction was allowed to continue at the same conditions until a TLC indicated a complete hydrolysis was accomplished. Pure XXXG-5ASA-NO₂ (105 mg) was obtained via reverse phase chromatography.

### Scheme 4, Step 4

XXXG-5ASA-NO₂ (82 mg) obtained in the previous step was dissolved in 7 mL of methanol/water (1:1). To this mixture was added 10 mg of Pd/C (10%) under stirring. Hydrogen was then introduced after degassing. The reduction was complete in 3 to 12 hrs. Pure product XXXG-5ASA (65 mg) was obtained via reverse phase chromatography (water/methanol, 90/10 to 50/50). The compound was characterized by proton NMR and mass spectrometry. m/z 1220 (M + Na⁺, C₄₆H₇₁NO₃₅Na requires 1220).

### EXAMPLE 2: In vitro Enzyme Kinetic Assays using XXXG-caged prodrugs

*In vitro* enzyme kinetic assays were performed on XXXG-5ASAME, XXXG-PRED, and XXXG-BUDE as examples of the glyco-caged prodrugs of the present application.

The hydrolysis of XXXG-PRED, XXXG-BUDE, XXXG-5ASAME and XXG-DEXA with the *Bacteroides ovatus* GH5 endo-xyloglucanase (Larsbrink J. et al. Nature 2014, see above) was demonstrated in sodium citrate buffer, pH 6.5, using a copper-bicinchoninic acid (BCA) reducing-sugar assay essentially as described in Amal G. et al. (2017) "A low-volume, parallel copper-bicinchoninic acid (BCA) assay for glycoside hydrolases", Chapter 1 in Protein-Carbohydrate Interactions: Methods and Protocols, Abbott D.W. and Lammerts van Bueren A. (eds.), Methods in Molecular Biology, 1588, 3-14. DOI:10.1007/978-1-4939-6899-2_1.

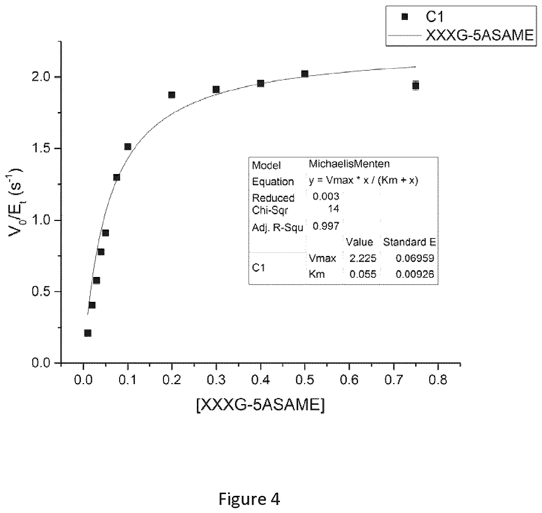

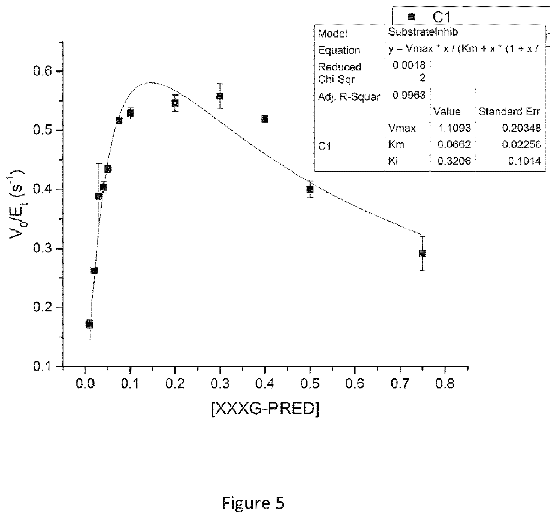

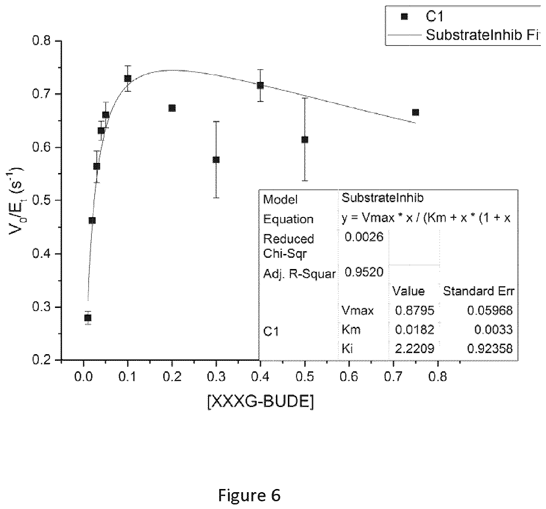

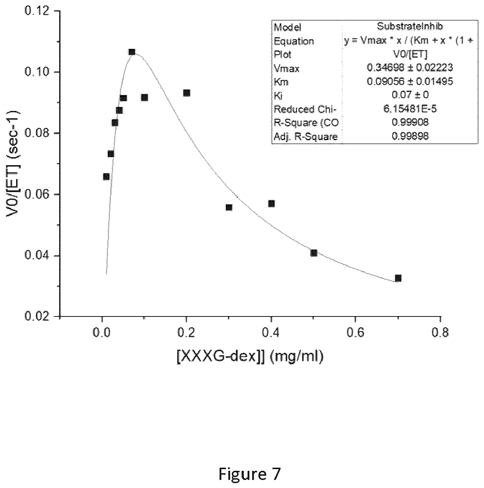

Results from the assay are summarized in Figures 4-7, and clearly demonstrated the release of the active drug in the presence of BoGH5A.

### EXAMPLE 3: Murine Study of XXXG-PRED and XXXG-DEXA Efficacy

### Study Description

This study was designed to directly compare the efficacy of steroids prednisolone and dexamethasone to their glyco-caged formulations (prepared according to embodiments of the present application) during intestinal inflammation. The bacterium, *Citrobacter rodentium* was administered to incite acute self-limiting colitis in mice. Germ-free mice were used in order to remove any confounding effects of the intestinal microbiota, and to purely observe the impacts of the steroid formulations during *C. rodentium* and *Bacteroides ovatus* co-colonization; *B. ovatus* colonization was necessary for drug de-caging.

The study was approved by the Animal Care Committee at the Agriculture and Agri-Food Canada (AAFC) Lethbridge Research and Development Centre (LeRDC), (Animal Use Protocol 1814).

The treatment groups represented in this experiment are listed below in Table 2.

**Table 2: Treatment groups**

| **Group** | ***Citrobacter rodentium*** | ***Bacteroides ovatus*** | **Treatment** (100 µL gavage) | **Sample number (*n*)** |
|---|---|---|---|---|
| **Germ-free control** | - | - | placebo (water) | 4 (histological scoring only) |
| **Control** | + | - | placebo (water) | 4 (histological scoring only) |
| **Control** | + | + | placebo (water) | 2 |
| **Pred^{a}** | + | + | prednisolone (1 mg/kg/day) | 2 |
| **Pred** | + | + | XXXG-PRED (1 mg/kg/day) | 2 |
| **Dex^{b}** | + | + | dexamethasone (0.6 mg/kg/day) | 2 |
| **Dex** | + | + | XXXG-DEXA (0.6 mg/kg/day) | 2 |

| | | | | |
|---|---|---|---|---|
| ^{a}Prednisolone ^{b}Dexamethasone | | | | |

### Materials & Methods

### Animals

Mice were reared from an germ-free (GF) C57BL/6 breeding colony at AAFC LeRDC, and ten males aged 8-months were used in this experiment. The gnotobiotic mice originally used to establish the GF breeding colony were purchased from the University of North Carolina at Chapel Hill (germ-free status C57BL/6J⁺). Mice were pair-housed (according to treatment) in sterilized Green Line Individually Ventilated Cage (IVC) Sealsafe PLUS^{™} mouse cages (Tecniplast) using axenic and containment level 2 procedures (Lange M.E. et al. 2019. "Housing gnotobiotic mice in conventional animal facilities". Curr. Protoc. Mouse Biol. e59). GF mice exitted the isolators and acclimatized to their environment for 3 days in IVCs with *ad libitum* access to AIN-93G + casein diet and 0.25% xyloglucan (XG) drinking water. The diet was prepared by Dyets Inc (Bethlehem) and the compostion in described in Table 3. The animal room was maintained on a 12-hr light/dark cycle at 22 °C.

**Table 3. Composition of AIN-93G Purified Rodent Diet***

| **Ingredient** | **kcal/g** | **g/kg** | **kcal/kg** |
|---|---|---|---|
| Casein | 3.6 | 200.0 | 716.0 |
| L-cystine | 4.0 | 3.0 | 12.0 |
| Sucrose | 4.0 | 100.0 | 400.0 |
| Cornstarch | 3.6 | 397.5 | 1430.9 |
| Dextrose | 3.8 | 132.0 | 501.6 |
| Soybean oil | 9.0 | 70.0 | 630.0 |
| t-butylhydroquinone | 0.0 | 0.014 | 0.0 |
| Cellulose | 0.0 | 50.0 | 0.0 |
| Mineral mix #210025 | 0.9 | 35.0 | 30.8 |
| Vitamin mix #310025 | 3.9 | 10.0 | 38.7 |
| Choline bitartrate | 0.0 | 2.5 | 0.0 |
| Total | | 1000.0 | 3760.0 |

| | | | |
|---|---|---|---|
| *Reeves P.G. et al. 1993. "AIN-93 purified diets for laboratory rodents: final report of the American institute of nutrition ad hoc writing committee on the reformulation of the AIN-76A rodent diet". J. Nutr. Vol 123, 1939-1951 | | | |

### Inoculations

Following acclimatization, mice were gavage-inoculated with *Bacteroides ovatus* (ATCC 8483) grown at 34 °C under anaerobic conditions. The single *B. ovatus* inoculum concentration was 2.0 × 10¹⁰ CFU/100 µL. After 2 days of *B. ovatus* colonization, mice were inoculated with *Citrobacter rodentium* (ATCC 51459) through an edible format (delivered on a 1.5% agar medium containing 5% ground diet). Mice were inoculated with C. *rodentium,* grown at 37°C under aerobic conditions, over the span of 2 days; 8.1 × 10⁸ CFU/100 µL as the primary and 4.1 × 10⁸ CFU/100 µL as the secondary inoculation. The mice were colonized with *B. ovatus* for a total of 7 days, and had an active *C. rodentium* infection for 5 of those days.

### Steroid Administration

The average weight of the mice (27.9 g) was calculated on the second day of the *Citrobacter rodentium* infection, and this average weight was used to calculate the steroid concentrations for all treatments. The steroids were dissolved as best as possible in sterile water, and stored at 4 °C until needed. Steroids were administered by gavage in 100 µL volumes for 3 consecutive days at the same time each morning (24 hrs between treatments) for the final 3 days of the *C. rodentium* infection. Mice in the control groups (without drug intervention) received water by gavage in 100 µL volumes. Gavage needles for drug administration were coated in sucrose solution (1 g/mL, syringe-filter sterilized) as a refinement procedure. Table 4 summarizes the steroid treatment dosages.

**Table 4: Steroid Dosages**

| **Steroid** | **Dosage** | **Quantity provided to mice** |
|---|---|---|
| Prednisolone, 99% Supplier: Millipore Sigma Cat. No. P6004 | 1 mg/kg/day | 0.028 mg/day |
| XXXG-PRED | 1 mg/kg/day | 0.109 mg/day |
| Dexamethasone, 98%, Supplier: Alfa Aesar Cat. No. A17590 | 0.6 mg/kg/day | 0.017 mg/day |
| XXXG-DEXA | 0.6 mg/kg/day | 0.061 mg/day |

During this study, prednisolone did not fully dissolve in water, and it was necessary to rinse the gavage needle with 50 µL of water and feed it to the mice to ensure they received the full dosage.

### Fecal collections

Fecal collections were necessary to enumerate the two species of bacteria that the mice were inoculated with. Feces were first collected on the day the mice transferred from the germ-free (GF) isolator to individually ventilated cages (IVC), and plated on Columbia agar with 10% blood under aerobic and anaerobic conditions to confirm the GF status of mice. During the experimental period, fecal collections from the cages took place 2 days following the *Bacteroides ovatus* inoculation, and two days following the *Citrobacter rodentium* inoculation. Since the mice were pair-housed, fecal samples were pooled. After fecal sampling, mice were transferred to sterile IVCs to ensure fresh samples for the next collection. At the experiment endpoint, digesta was excised from the large bowel so that bacterial densities in individual mice could be determined. The bacterial populations were enumerated using microbiological culturing techniques; feces were serial diluted and plated on either Columbia agar to estimate a colony forming units (CFU)/g concentration of *B. ovatus* (grown under anaerobic conditions at 37 °C) or plated on MacConkey agar to estimated a CFU/g concentration of *C. rodentium* (grown under aerobic conditions at 37 °C).

### Necropsy

Under isoflurane anesthesia, mice were terminally bled by cardiac puncture and then humanely euthanized by cervical dislocation. All collected blood was separated into serum that was dedicated for steroid detection and optimization of steroid detection procedures. Following euthanasia, a midline laparotomy was made and gross observations of the abdominal regions were photo-documented. Approximately 1 cm sections of the cecum, and the most proximal and distal portions of the colon were divided for histological examination. Cecal and colonic tissues were flash frozen in liquid nitrogen for future immunological protein quantification. Digesta extracted from the colon was stored at -80 °C for future steroid detection, and fresh feces from the cages were used to enumerate *Bacteroides ovatus* and *Citrobacter rodentium* by microbiological techniques on the same day as the necropsy.

### Histopathology

Tissue samples (cecum, proximal colon, and distal colon) were initially stored for preservation in 10% buffered formalin. Tissue processing, sectioning, and staining with hematoxylin and eosin (H&E) were carried out using standard protocols. Histological scoring of inflammation was performed by a veterinary pathologist blinded by treatment, with scoring criteria from previously described methods (Barthold S.W. et al. 1978. "Transmissible murine colonic hyperplasia". Vet. Pathol. Vol 15: 223 - 236; and Wu X. et al. 2008. "Saccharomyces boulardii ameliorates Citrobacter rodentium-induced colitis through actions on bacterial virulence factors". Am. J. Physiol. Gastrointest. Liver Physiol. Vol 294: G295 - 306). The specific categories for scoring are presented in Tables 5-7 below. The total pathology scores illustrate the sum of scores for all histopathological categories observed for each mouse. The tissues analyzed for germ-free mice and *C. rodentium*-infected mice (i.e. without drug intervention) controls were collected from a previously conducted experiment (non-related). The mice were infected with *C. rodentium* by the same protocol, and these controls were required to visually examine the comparative histological differences in the absence of both *Bacteroides ovatus* and steroid intervention. It was not possible to conduct the experiment with these control treatments as only a limited number of male germ-free mice were available.

### Tissue Cytokine Detection

Cecal and colonic homogenate for each mouse was prepared using the tissues suspended in an immunoprecipitation buffer at a ratio of 1:5 (wt/vol, adapted from Burgos-Ramos E. et al. 2012. "Adipose tissue promotes a serum cytokine profile related to lower insulin sensitivity after chronic central leptin infusion". PLoS One Vol 7(10): e46893) with the addition of 1% Protease Inhibitor Cocktail (Sigma Aldrich). Frozen tissue sections were immediately homogenized using a Tissue-Tearor^{®} model 398 homogenizer (Biospec Products) and centrifuged (14 000 × g for 30 min at 4 °C). Supernatants were collected and used immediately for cytokine profiling. Cecal and colonic cytokine concentrations were measured for each treatment group by enzyme-linked immunosorbent assay (ELISA). Cytokines included in the analysis were interleukin-1 beta (IL-1β); IL-6; IL-10; IL-22; and tumour necrosis factor alpha (TNF-α). All cytokines were quantified using mouse DuoSet ELISA Development kits according to the manufacturer's protocols (R&D Systems). ELISAs were performed using 96-well high-binding half area microplates (Greiner Bio-One), and the optical density of the reactions were determined at wavelength of 450 nm on a Synergy HT^{™} multi-detection microplate reader (BioTek Instruments Inc.) with Gen5^{™} analysis software (BioTek Instruments Inc.).

### Results

### Body Weight, Gross Morphology and Histologic Changes

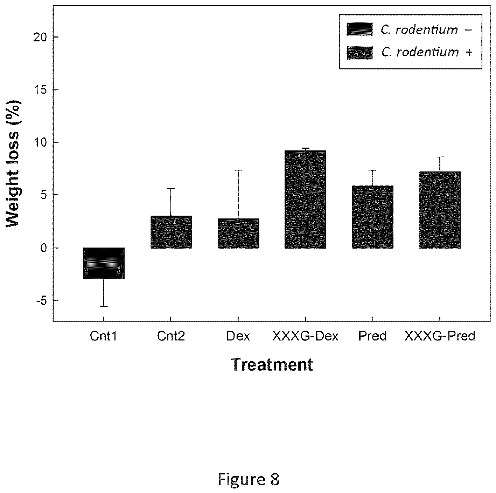

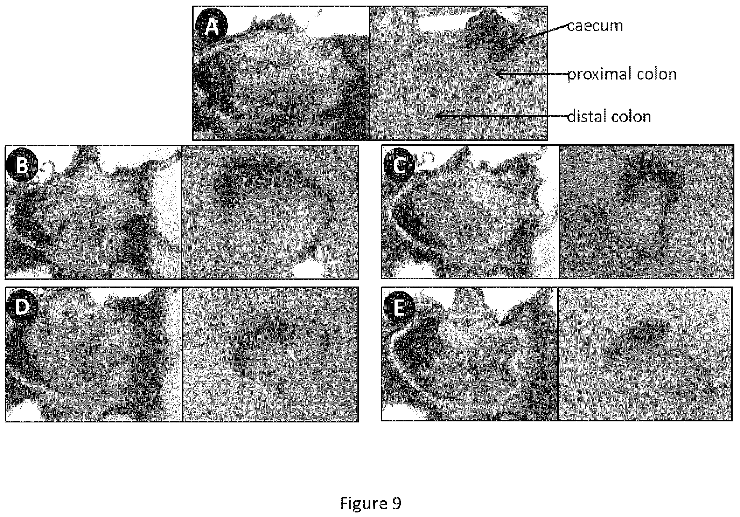

There were no treatment effects on weight loss in mice acutely infected with *Citrobacter rodentium* after 5 days (Figure 8), and all mice remained at a healthy weight. There was evidence of gross histopathologic changes in mice as a result of steroid administration (Figure 9). Visually, the XXXG-DEXA treatment displayed less cecal and colonic thickening than uncaged dexamethasone, and both XXXG-PRED and uncaged prednisolone treatments had more blood present in ceca than dexamethasone treatment.

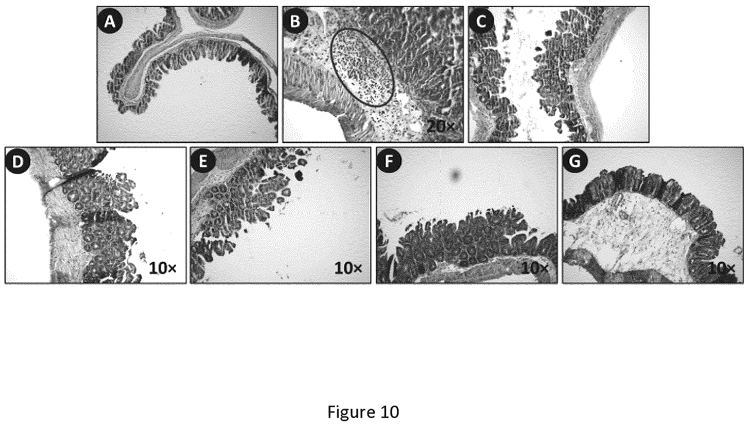

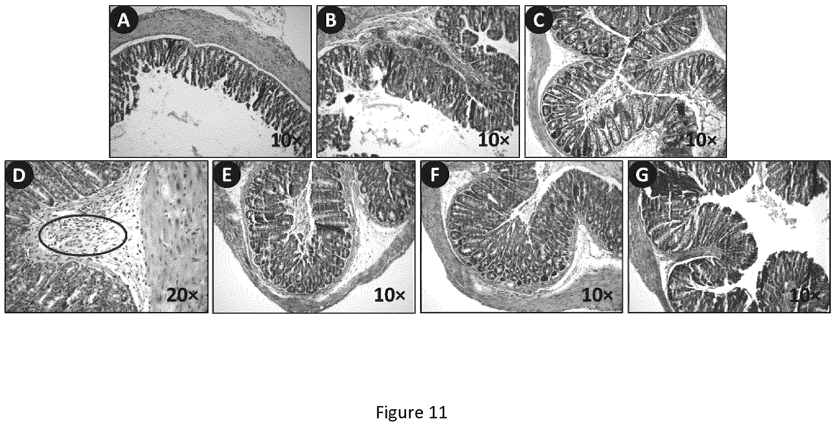

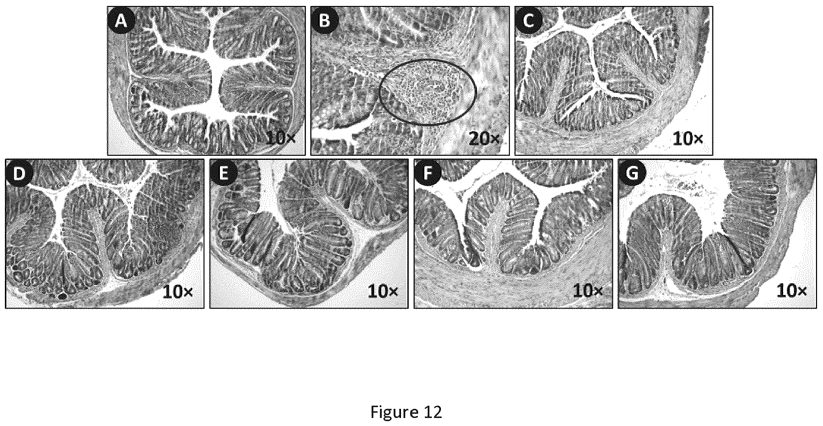

Histologic examination was performed on cecal (Table 5, Figure 10), proximal colonic (Table 6, Figure 11), and distal colonic samples (Table 7, Figure 12). The total scores were calculated as the sum of each scoring category. To generalize, XXXG-DEXA treated ceca and proximal colon tissues displayed less injury and inflammation intensity than the other treatments. The conjugated steroids XXXG-DEXA and XXXG-PRED had equal scores in the distal colon, and lessened the injury and inflammation intensities in this tissue.

**Table 5: Hisotological scoring of cecal tissue**

| **Treatment** | ***n*** | **Epithelia Hyperplasia** | **Crypt Height** | **Epithelial Injury** | **Inflamm** | **Globlet cell** | **Mitotic Act.** | **Fibrosis** | **Total Score** |
|---|---|---|---|---|---|---|---|---|---|
| **GF Control** | 4 | 0.25 | 0 | 0 | 0 | 0 | 0 | 0.25 | 0.5 |
| ***C*. *rodentium* control** | 4 | 2.75 | 2.75 | 2.5 | 3.75 | 0.75 | 1.75 | 2 | 16.25 |
| ***C*. *rodentium* & *B. ovatus* control** | 2 | 0 | 0 | 1 | 1 | 0 | 0 | 1 | 3 |
| **dexamethasone** | 2 | 1 | 1 | 0.5 | 2 | 0 | 1.5 | 0 | 6 |
| **XXXG-DEXA** | 2 | 1 | 1 | 1 | 2 | 0.5 | 1.5 | 1.5 | 8.5 |
| **prednisolone** | 2 | 1.5 | 1.5 | 1 | 1.5 | 0.5 | 1 | 1 | 8 |
| **XXXG-PRED** | 2 | 2 | 2 | 1.5 | 2.5 | 0 | 1.5 | 0.5 | 10 |

**Table 6: Hisotological scoring of the proximal colon**

| **Treatment** | ***n*** | **Epithelia Hyperplasia** | **Crypt Height** | **Epithelial Injury** | **Inflamm** | **Globlet cell** | **Mitotic Act.** | **Fibrosis** | **Total Score** |
|---|---|---|---|---|---|---|---|---|---|
| **GF Control** | 4 | 0.5 | 0.25 | 0.75 | 0.5 | 0 | 1 | 0.75 | 3.75 |
| ***C. rodentium* control** | 4 | 1.5 | 1.75 | 2 | 2.75 | 0.5 | 1.75 | 1 | 11.25 |
| ***C. rodentium & B. ovatus* control** | 2 | 2.5 | 2 | 2.5 | 2 | 0 | 2 | 1 | 12 |
| **dexamethasone** | 2 | 3 | 3 | 2 | 1.5 | 0 | 1.5 | 0 | 11 |
| **XXXG-DEXA** | 2 | 1.5 | 2 | 2 | 1 | 0 | 1.5 | 0 | 8 |
| **prednisolone** | 2 | 1.5 | 2 | 2 | 1.5 | 0.5 | 1.5 | 0 | 9 |
| **XXXG-PRED** | 2 | 3 | 2 | 2.5 | 2 | 0.5 | 1.5 | 0 | 11.5 |

**Table 7: Hisotological scoring of the distal colon**

| **Treatment** | ***n*** | **Epithelia Hyperplasia** | **Crypt Height** | **Epithelial Injury** | **Inflamm** | **Globlet cell** | **Mitotic Act.** | **Fibrosis** | **Total Score** |
|---|---|---|---|---|---|---|---|---|---|
| **GF Control** | 4 | 0.75 | 0.25 | 0.75 | 0.75 | 0 | 0.5 | 0.25 | 3.25 |
| ***C. rodentium* control** | 4 | 3 | 2.75 | 2.5 | 3.5 | 1.5 | 2 | 1.75 | 17 |
| ***C. rodentium & B. ovatus* control** | 2 | 2.5 | 3 | 2.5 | 1.5 | 0 | 2 | 0 | 11.5 |
| **dexamethasone** | 2 | 1.5 | 2 | 1 | 1.5 | 1.5 | 1 | 1 | 9.5 |
| **XXXG-DEXA** | 2 | 1.5 | 1.5 | 1 | 1 | 0 | 1 | 0 | 6 |
| **prednisolone** | 2 | 1.5 | 1.5 | 2 | 1.5 | 0.5 | 1.5 | 1.5 | 10 |
| **XXXG-PRED** | 2 | 1.5 | 1.5 | 1 | 1 | 0.5 | 1 | 0 | 6.5 |

### Bacterial Densities

The gastrointestinal tract was initially colonized by *Bacteroides ovatus,* however, densities were reduced 8 days post-inoculation. Without wishing to be bound by theory, this could have been due to either natural clearance of *B. ovatus* or competitive exclusion by *Citrobacter rodentium,* and was not related to treatment. The densities of *C*. *rodentium* were considered equivalent in the fecal content regardless of steroid treatment.

### Cecal and Colonic Cytokines

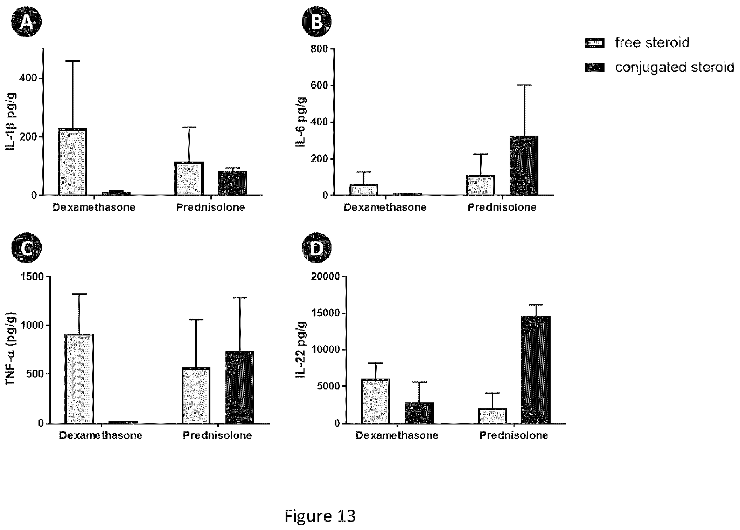

Cecal cytokine concentrations were measured from tissue homogenates (Figure 13). Both XXXG-DEXA and XXXG-PRED decreased concentrations of pro-inflammatory cytokines IL-1β and TNF-α, and XXXG-DEXA was more effective than XXXG-PRED in decreasing the concentration of pro-inflammatory cytokine IL-6 when compared to the free steroids. XXXG-PRED increased the concentrations of IL-22 in the cecal tissue, which is an important Th17 cytokine with involvement in would healing and tissue regeneration.

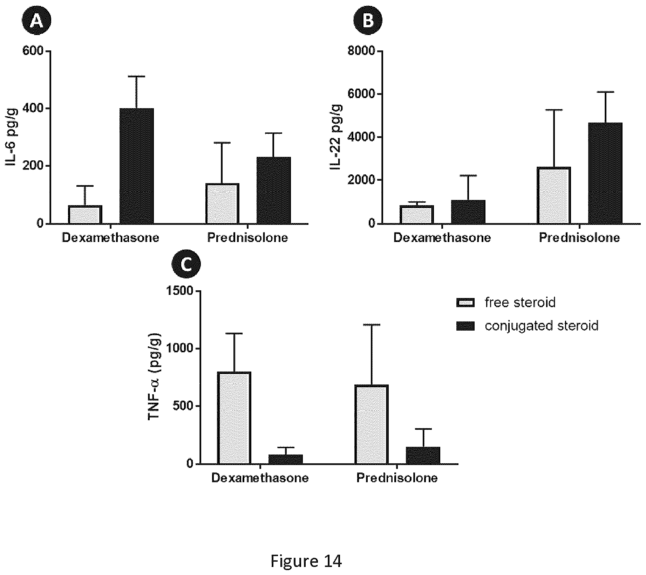

Colonic cytokines were also measured from tissue homogenates (Figure 14). The glyco-caged prodrugs did not lower concentrations of pro-inflammatory cytokine IL-6 in the colonic tissue as they did in the cecal tissue. Similar to the cecal tissue, the gyco-caged prodrug treatments lowered concentrations of pro-inflammatory cytokine, TNF-α, and also increased concentrations of the anti-inflammatory cytokine, IL-22 in colonic tissues.

### Conclusions

Histopathologically, XXXG-DEXA and XXXG-PRED were found to ameliorate acute inflammation in the proximal and distal colon of gnotobiotic mice colonized by *B. ovatus.* XXXG-DEXA exhibited a greater efficacy than uncaged dexamethasone. XXXG-PRED also exhibited a greater efficacy than uncaged prednisolone.

XXXG-DEXA was more effective than XXXG-PRED in ameliorating colitis in the cecum and proximal colon incited by *Citrobacter rodentium,* and both glyco-caged steroid formulations were equally efficacious in the distal colon.

XXXG-DEXA and XXXG-PRED were more effective than the uncaged drugs in decreasing pro-inflammatory cytokines concentrations (IL-1β and TNF-α), and increasing the concentration of the anti-inflammatory, IL-22 in intestinal tissues. There were differential impacts on IL-6 concentrations in cecal and colonic tissues.

Overall, the results of this study demonstrate that the glyco-caged prodrugs of the present application are effective in providing active drug to a subject and in treatment of an enteric inflammatory disease in a subject, at the same or better efficacy than the corresponding uncaged drug.

EXAMPLE 4: Synthesis of the Self-Immolative Linker-Containing Glyco-Caged Prodrug, XXXG-*β*-O- SIL-mesalazine (XXXG-SIL-5ASA)

The of XXXG-SIL-SASA (see Table 8 below) is described in Scheme 5, beginning from readily available powdered tamarind seed kernel powder. Where problems may arise due to functional group compatibilities to the reagents used, these functional groups were protected with an appropriate protecting group by a known method in advance. These protecting groups were subsequently removed by known methods. The reaction conditions were optimized to increase the combined overall yield and/or to minimize environmental impacts.

**Table 8: Self-immolative glyco-caged prodrug**

| **Compound** | **Abbreviation** | **Chemical Structure** |
|---|---|---|
| XXXG-*β*-O-SIL-mesalazine | XXXG-SIL-SASA | 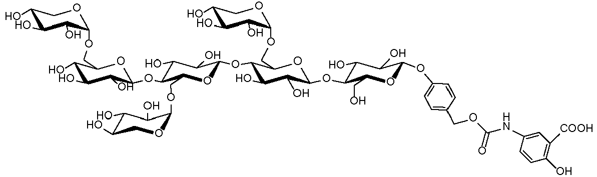 |

### Synthesis of XXXG-SIL-5ASA

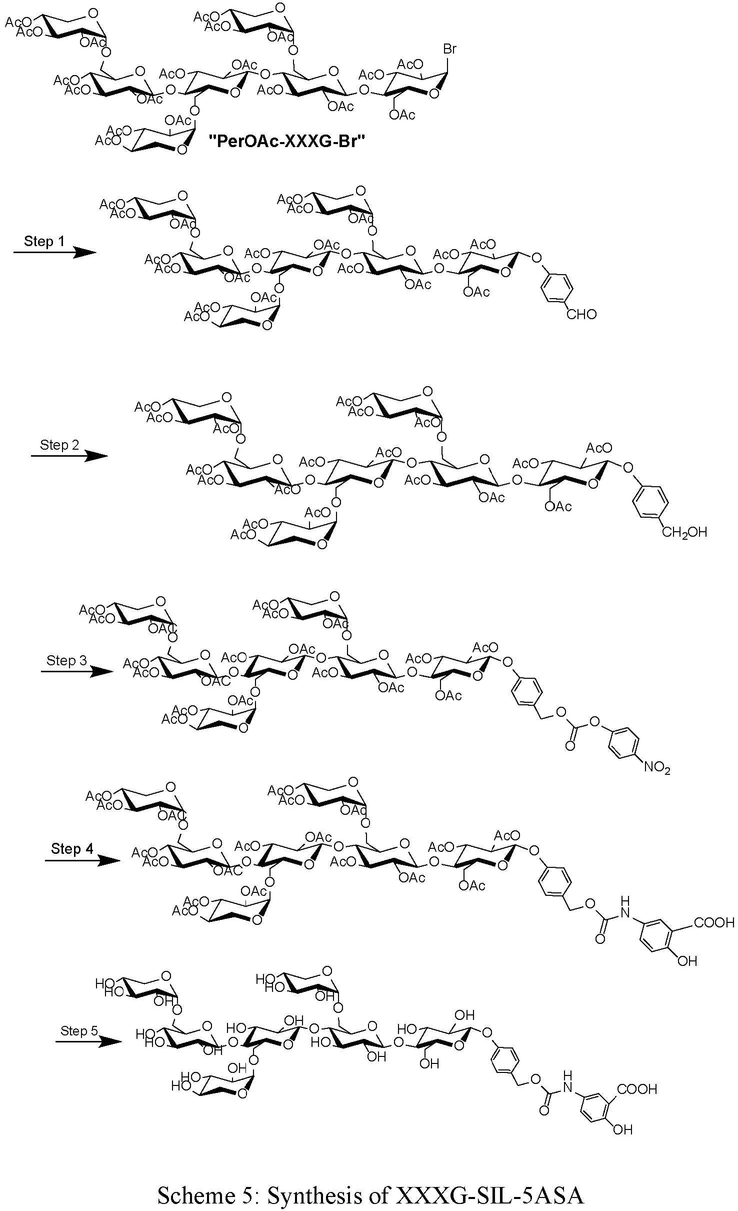

### Scheme 5, Step 1

Per-O-Ac-XXXG-Br (154 mg) and 4-hydroxybenzaldehyde (214 mg) was dissolved in 8 mL of dry acetonitrile under stirring, followed by the addition of 0.94 g of 3Å MS and 250 mg of Ag₂O. The suspension was then stirred in the dark for 24 hrs before all the volatiles were removed under reduced pressure. The crude thus obtained was purified with flash silica gel chromatography (toluene/acetone 4:1 to 2:1). The conjugated product Per-O-Ac-XXXG-4'-O-benzaldehyde (75 mg) was obtained as a white powder. m/z 1987 (M + Na⁺, C₈₄H₁₀₈NO₅₃Na requires 1987).

### Scheme 5, Step 2

Per-O-Ac-XXXG-4'-O-hydroxybenzaldehyde (75 mg), silica gel (0.8 g), and IR-120 resin (H+ form, 16 mg) were suspended in chloroform and IPA (6 mL 5:1) under argon at 0 °C. 16 mg of NaBH₄ was added over a period of 30 mins. The reaction was allowed to continue until a TLC test indicated a complete reduction. The reaction was quenched with saturated ammonium chloride, followed by the addition of 120 mL of ethyl acetate and water (1:1). The organic phase was separated and washed with water (60 mL X 3) and brine (50 mL X 2) before it was concentrated under reduced pressure. The crude thus obtained was purified with flash chromatography (toluene/acetone 4:1 to 2:1) and Per-*O*-Ac-XXXG-4'-*O*-benzylalcohol (65 mg) was obtained as a pure product as evidenced by proton NMR and MALDI mass spectrometry. m/z 1989 (M + Na⁺, C₈₄H₁₁NO₅₃Na requires 1989).

### Scheme 5, Step 3

Per-O-Ac-XXXG-4'-O-benzylalcohol (33.5 mg) was dissolved in 6 mL of DCM under argon at 0 °C, followed by the addition of 42 mg of pyridine and 82 mg of p-nitrophenylchloroformate. The resultant mixture was stirred at the same conditions for 2 hrs before the volatiles were removed under reduced pressure. The crude thus obtained was purified with flash chromatography (toluene/acetone 4:1 to 2:1) and Per-*O*-Ac-XXXG-4'-nitro-benzylcarbonate (32.1 mg) was obtained as a pure product as evidenced by proton NMR and MALDI mass spectrometry. m/z 2154 (M + Na⁺, C₉₁H₁₁₃NO₅₇Na requires 2154).

### Scheme 5, Step 4

Per-O-Ac-XXXG-4'-nitro-benzylcarbonate (25.5 mg) was dissolved in 10 mL of DCM at 0 °C, followed by the addition of 6 mg of 5-ASA and 55 mg of DMAP. The reaction mixture was stirred for 0.5 hr before it was allowed to warm to room temperature. The reaction mixture was stirred for additional 2 hrs until a TLC indicated a complete reaction. 0.5 g of silica gel added and the resultant suspension was concentred under reduced pressure. The resultant crude was subject to a flash silica gel chromatography (DCM/acetonitrile/triethylamine) and 25 mg of Per-O-Ac-XXXG-SIL-5 ASA was obtained. m/z 2168.7 (M + Na⁺, C₉₂H₁₁₅NO₅₇Na requires 2168.6).

### Scheme 5, Step 5

Per-*O*-Ac-XXXG-SIL-5ASA (25 mg) was dissolved in 4.5 mL of MeOH/DCM (2:1). Catalytic amount of sodium methoxide (2 mg) was added and the resultant suspension was stirred overnight before all volatiles were removed under reduced pressure at 40 °C. The crude thus obtained was subject to reverse phase chromatography. 12 mg of XXXG-SIL-SASA was obtained as evidenced by proton NMR and mass spectrometry. m/z 1392.3973 (M - H⁺ + 2 Na⁺, C₅₄H₇₆NO₃₈Na₂ requires 1392.3841).

### EXAMPLE 5: Synthesis of the Self-Immolative Linker-Containing Glyco-Caged Prodrug, XXXG-β-O- SIL-peptide (XXXG-SIL-peptide)

The synthesis of XXXG-SIL-peptide (see Table 9 below) is described in Scheme 6, beginning from the common intermediate Per-O-Ac-XXXG-4'-nitro-benzylcarbonate. The reaction conditions were optimized to increase the combined overall yield and/or to minimize environmental impacts.

**Table 9: Self-immolative glyco-caged prodrug**

| **Compound** | **Abbreviation** | **Chemical Structure** |
|---|---|---|
| XXXG-*β*-O-SIL-peptide | XXXG-SIL-peptide | 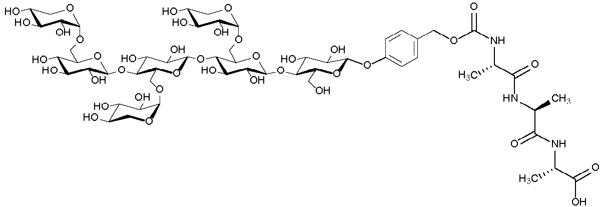 |

### Synthesis of XXXG-SIL-peptide

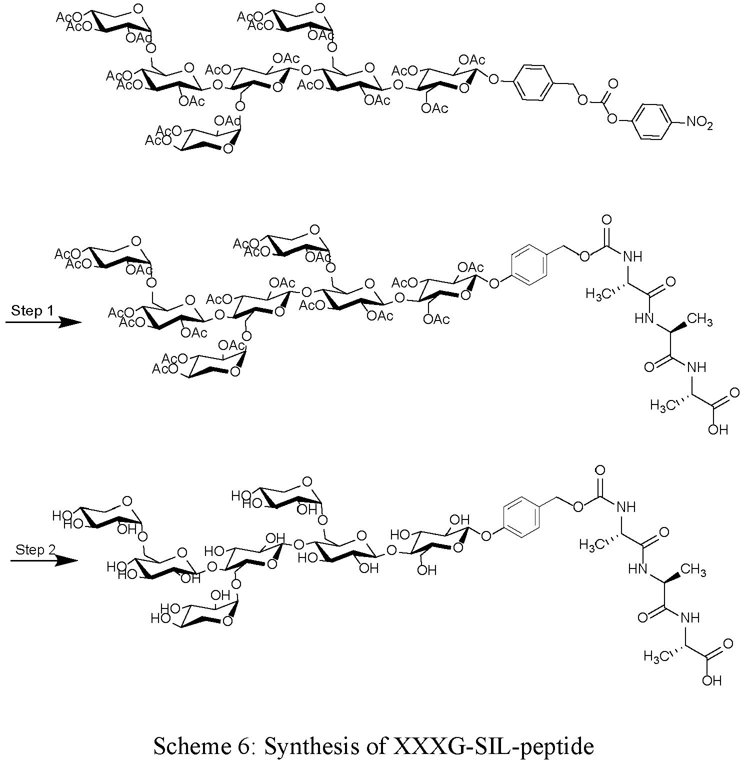

### Scheme 6, Step 1

Per-O-Ac-XXXG-4'-nitro-benzylcarbonate (50 mg) was dissolved in 10 mL of DCM at 0 °C, followed by the addition of 100 mg of Alanine Tripeptide and 120 mg of DMAP. The reaction mixture was stirred for 0.5 hr before it was allowed to warm to room temperature. The reaction mixture was stirred for additional 2 hrs until a TLC indicated a complete reaction. 0.7 g of silica gel added, and the resultant suspension was concentred under reduced pressure. The resultant crude was subject to a flash silica gel chromatography (DCM/acetonitrile/triethylamine) and 45 mg of Per-0-Ac-XXXG-SIL-Peptide was obtained as evidenced by MALDI mass spectrometry. m/z 2246 (M + Na⁺, C₉₄H₁₂₅N₃O₅₈Na requires 2246).

### Scheme 6, step 2

Per-O-Ac-XXXG-SIL-Peptide (45 mg) was dissolved in a mixture of methanol and dichloromethane (6 mL, 4: 1) at room temperature under stirring. To this mixture 7.5 µL of a 25% (w/w) solution of sodium methoxide in methanol was added and the resultant suspension was stirred for 6 hrs until a TLC test indicated the complete consumption of the starting material. 200 mg of Amberlite^{™} IR-120 resin (H+ form) and stirred for 10 min, whereupon the suspension was filtered, and the filtrate was concentrated under reduced pressure and a 20 mg of XXXG-SIL-Peptide was obtained as evidenced by thin layer chromatography.

This example demonstrates the successful incorporation of a tri-alanine peptide as a model drug moiety in a glyco-caged prodrug. Although the tri-alanine peptide does not have any known effect on IBD or other intestinal disorders this example clearly shows that N-terminal amines on peptides are readily conjugated to an intermediate useful in the production of the self-immolative linker-containing glyco-caged prodrugs described herein. Accordingly, this example shows that peptides, and polypeptides, having free amine groups (for example in alanine and lysine side chains) can be successfully incorporated into self-immolative linker-containing glyco-caged prodrugs.

### EXAMPLE 6: Decaging of Glyco-caged Prodrugs by Xyloglucanases from Enteric Bacteria

The glyco-caged prodrugs herein are "decaged," or metabolized to release pharmacologically active prodrugs, by the action of xyloglucanases. Such xyloglucanases are found in bacteria present the intestine of animals.

The present example demonstrates that xyloglucanase-active bacteria are present in mice, chickens and in the large intestine of swine.

### Bacterial strains from pig intestines

Approval to conduct this project was obtained from the AAFC LeRDC Animal Care Committee (Animal Use Protocol 1512). Castrated male piglets at 6-weeks of age provided a mini-pellet ration diet that was free of antibiotics (Proform Pig Starter 2). Feed was provided daily, and piglets were permitted to eat and drink *ad libitum.* Half of the piglets were orally inoculated with *Salmonella enterica* Typhimurium DT104 (SA970934) in buffer to incite enterocolitis, and the remaining half were orally administered buffer alone. At 2, 6, and 10 dpi, randomly designated animals were anesthetized for sample collection from live animals. Piglets were sedated, intubated, and general anesthesia established with isoflurane. From animals under general anesthesia, segments of intestine were collected. To ensure the integrity of the intestinal segment and to minimize release of ingesta, double ligatures were established at the two ends of the segment, and the segment was excised from the intestine by cutting between the two ligatures. Bacteria were isolated using the basic method described in Moote P.E. et al. (2020. "Application of culturomics to characterize diverse anaerobic bacteria from the gastrointestinal tract of broiler chickens in relation to environmental reservoirs". Can. J. Microbiol. Vol 66: 288 - 302). Isolated bacteria were identified by sequencing the partial 16S rRNA gene. To screening bacteria for endoxyloglucanase activity, congo red staining was used. Briefly, isolated bacteria were grown on Columbia Agar supplemented with 10 mL/L of 0.05% Hemin and xyloglucan (0.25%) in gridded dishes, and cultures were incubated anaerobically for 24-48 hrs. Cultures were removed from the chamber, and 15 mL of 0.1 M phosphate buffered saline (pH 7) was added, bacterial biomass was scraped from the surface of the medium, and the liquid was removed. Clutures were then stained with 1.0% congo red at room temperature for 20 min, the congo red solution was removed, cultures were flooded with 1 M sodium chloride for 20 min, the salt solution was removed, and the medium was fixed with 1 M HCl to produce the blue stained medium with zones of clearing indicative of xyloglucanase activity. Cultures were photodocumented. for evaluations and documentation.

The following strains of *Bacteroidetes* bacteria that were capable of growth on xyloglucan and exhibited clearing of the medium indicative of endo-xyloglucanase activity (i.e. using the congo red medium) were isolated from pig intestines:

| **Strain No.** | **Identity** |
|---|---|
| 3 | *Bacteroides uniformis* |
| 5 | *Bacteroides uniformis* |
| 11 | *Bacteroides uniformis* |
| 12 | *Bacteroides uniformis* |
| 17 | *Bacteroides uniformis* |
| 20 | *Bacteroides uniformis* |
| 37 | *Bacteroides uniformis* |
| 40 | *Bacteroides uniformis* |
| 58 | *Bacteroides uniformis* |
| 59 | *Bacteroides uniformis* |
| 60 | *Bacteroides uniformis* |
| 92 | *Parabacteroides merdae* |

XXXG-resorufin was synthesized and used as a model glyco-caged prodrug substrate used to facilitate monitoring of decaging. In particular, cleavage of the XXXG-resorufin substrate was used to measure the xyloglucanase activity of bacterial populations and communities isolated or extracted from pigs.

A limited number of the additional bacterial taxa isolated from the intestines of pigs exhibited endo-xyloglucanase activity, and are thus likely to be capable of decaging XXXG-resorufin (e.g., *Streptococcus gallolyticus*)*.* Thus, decaging ability within the intestine is not restricted to *Bacteroides* species and related bacteria. Importantly, evidence of bacterial abundance indicated that *B. uniformis* and *S. gallolyticus* were differentially associated with inflamed intestine, which may further enhance targeting of prodrugs at sites of inflammation (Bescucci D.M. *et al. unpublished*)*.*

To screen the xyloglucan degrading activity, bacterial isolates were screened and *Bacterioides ovatus* wild type and the ΔGH5 deletion mutant were used as positive and negative controls, respectively. Briefly, the strains stored in glycerol at -80 °C were recovered by inoculation into rich Columbia medium and cultivated anaerobically at 37 °C for 24 hrs. Next, the cells were pelleted by centrifugation 3,000 x g and the supernatant was decanted, followed by washing the cells twice with minimum medium supplemented with chopped meat. The pellets were re-suspended in 1 mL of the minimum medium, and 100 µL of the suspension was transferred to a culture tube pre-filled with 1.5 mL of the minimum medium containing either high purity xyloglucan (Megazyme International Ireland Ltd.), D-glucose (0.5% w/v), or no carbohydrate, followed by anaerobic incubation at 37 °C for 24 hrs. The optical density at a wavelength of 600 nm (OD600) was then measured for each bacterial suspension. Half millilitre of each suspension with (i.e., lysed) or without (i.e., whole cells) pre-incubated with BugBuster^{™} lysis reagent (Novagen, EMD Chemicals Inc.) was mixed with 0.5 mL of the minimum medium containing 100 µmol/L of XXXG-resorufin followed by incubation of the mixture for 4 hrs at room temperature with tubes fully covered with aluminium foil. After the incubation, the cells were heat-killed at 90 °C for 5 min before centrifuging the tubes and transferring 200 µL of each supernatant to a 96-well plate. Fluorescence signal was collected from the plate on a BioTek Synergy^{™} HT multi-detection microplate reader (BioTek Instruments) installed with a 530/25 nm excitation filter and a 590/35 nm emission filter, and the data were analyzed using the Gen5 2.04 software. Relative fluorescence unit (RFU) was calculated as the ratio of fluorescence reading (with the subtraction of background fluorescence) to OD600.

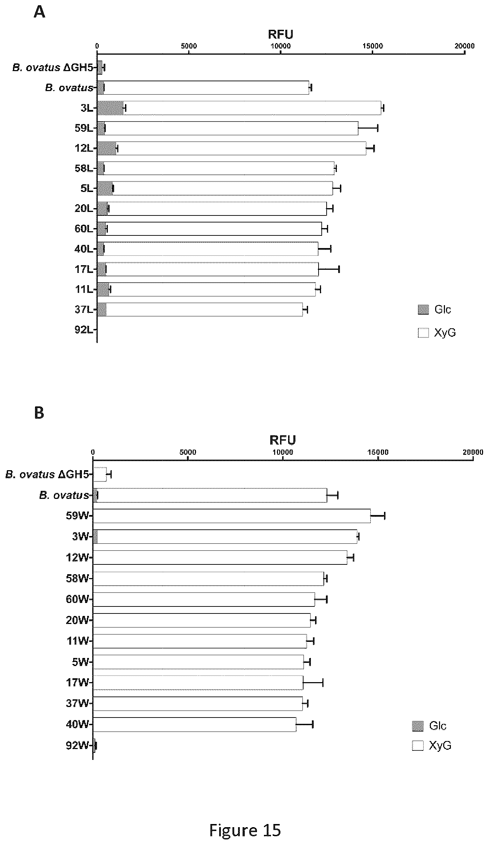

Figure 15A shows the decaging activity of whole cells of the above strains on XXXG-resorufin, in comparison to *B. ovatus* as a positive control and mutant *B. ovatus* lacking a xyloglucanase (ΔGH5). Figure 15B shows the decaging activity of lysed cells of the above strains on XXXG-resorufin, in comparison to *B. ovatus* as a positive control and mutant *B. ovatus* lacking a xyloglucanase (ΔGH5). The bacteria were grown on either glucose or xyloglucan as the sole carbon source. As illustrated, the *Bacteroides uniformis* strains were all able to decage XXXG-resorufin indicating the presence of endo-xyloglucanase in these strains.

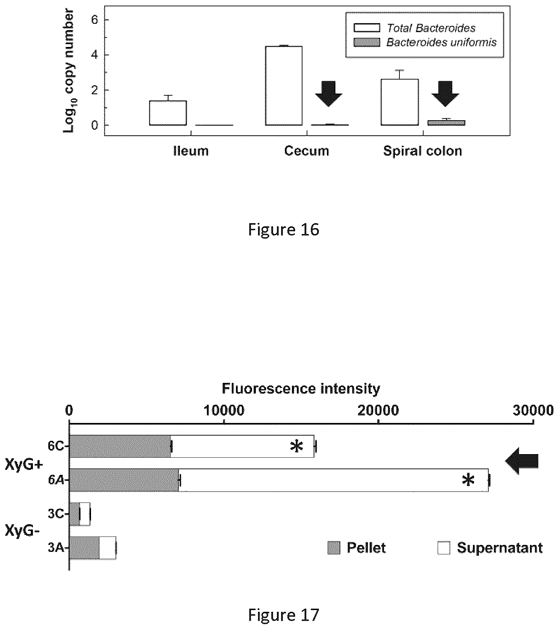

The distribution of *Bacteroides* in the gastro-intestinal tract of pigs is illustrated in Figure 16. As shown, strains of *Bacteroides* are present in the ileum, cecum and in the spiral colon, but *B. uniformis* was found only in the cecum and colon.

A study was performed using a pig colon bioreactor, to demonstrate that XXXG-resorufin could be decaged under *ex vivo* conditions to mimic *in vivo* conditions, with or without the addition of xyloglucan. The intestinal tract of a pig was obtained from a local abattoir, and within 30 min of death, ligatures were established as descrived above, and segments of the colon were excised thereby ensuring that air did not infiltrate the segments. The segments were transferred to an anaerobic chamber with a N₂ predominant atmosphere, and digesta was harvested from the segments within the chamber. The digesta was used to populate bioreactor vessels. The basic bioreactor methodology described by Auchtung J.M. et al. (2016. "MiniBioreactor Arrays (MBRAs) as a tool for studying C. difficile physiology in the presence of a complex community". Meth. Mol. Biol. Vol 1476: 235 - 250) was followed. Half of the reactors were fed medium amended with xylogucan (0.33 g/L) and the remaining vessels were fed medium alone. An aliquot (0.5 mL) of the bioreactor contents were centrifuged at 3,000 x g to fractionate the pellet from the supernatant. The pellet was resupended in 0.5 mL of sterile bioreactor medium. Both the supernatant and the pellet solution were incubated with 0.5 mL of 30 µmol/L XXXG-resorufin. The fluorescence assay was performed as descrbed above. As shown in Figure 17, there was substantive induction of xyloglucanase activity and decaging of XXXG-resorufin when xyloglucan is added to the bioreactor (XyG+). In contrast, there was only low decaging activity without induction by xyloglucan (XyG-). The significant extracellular activity (shown in the supernatant) suggests that at least a portion of the xyloglucanase(s) is secreted from the bacteria. Results show that bacteria with decaging ability exist naturally in the intestinal tract of pigs.

### Bacterial strains from chicken intestines

Approval to conduct this project was obtained from the AAFC LeRDC Animal Care Committee (Animal Use Protocol 1622). Ross 308 broiler chickens were used. Chicks (1-day-old) were obtained from a local hatchery, and were reared within two stainless steel pens (2.23 m² per pen with 10 birds per pen for a stocking density of 0.22 m² per bird). Birds were provided free access to a non-medicated starter diet (Hi-Pro Feeds) and water. Ambient room temperature was maintained at 22 ± 2°C, and birds were provided free access to brooders for the first 20 days. At 31-days-of-age, arbitrarily-selected birds were anesthetized with isoflurane and humanely euthanized. Bacteria were isolated from the ceca of birds using a variety of anaerobic isolation methods as described in Moote P.E. et al. (2020. "Application of culturomics to characterize diverse anaerobic bacteria from the gastrointestinal tract of broiler chickens in relation to environmental reservoirs". Can. J. Microbiol. Vol 66: 288 - 302). Isolated bacteria were identified by sequencing the partial 16S rRNA gene. Two different isolates of *Bacteroides ovatus* that were isolated from the ceca of broiler chickens demonstrated their ability to decage XXXG-resorufin to generate fluorescent resorufin.

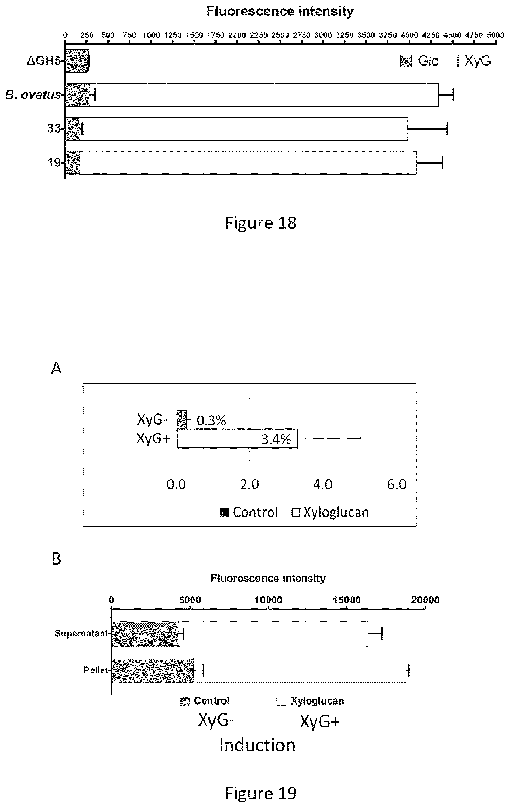

The two isolates of *Bacteroides ovatus* (33 and 19) isolated from the ceca of chickens were cultured on glucose or xyloglucan and incubated with XXXG-resorufin. The results are shown in Figure 18; both isolates exhibited endo-xyloglucanase activity and were able to effectively decage the XXXG-resorufin. The positive control was previously isolated *B*. *ovatus* and the negative control was the mutant *B. ovatus* lacking a xyloglucanase (ΔGH5) as used above. These results show that bacteria with decaging ability exist naturally in the intestinal tract of chickens.

### Xyloglucanase activity in mouse feces

Approval to conduct this project was obtained from the AAFC LeRDC Animal Care Committee (Animal Use Protocol 1924). To demonstrate the presence of naturally occurring bacteria having xyloglucanase activity in mice, feces from C57BL/6 mice maintained on an AIN-93 G basal diet were collected. One half of the mice were provided the basal diet (XyG-), and the other half of the mice were maintained on the basal diet amended with 2% xyloglucan (XyG+). Freshly excreted feces were obtained from both groups. A portion of the feces was used to isolate anaerobic bacteria possessing endoxyloglucanase activity, and the remaining portion was used to quantify decaging of XXXG-resorufin. Xyloglucanase positive colony forming units (CFUs) were visualized using the congo red screening method described above, and CFUs that produced a zone of clearing were enumerated and isolated. The clearing ability of isolated bacteria was confirmed as above.

To assess the ability of fecal bacteria to decage XXXG-resorufin, 0.5 mg of feces were homogenized in 5.0 mL of sterile buffer and spun at 3,000 x g. The soluble fraction, represented lysed cells and secreted enzymes; and the pellet resuspened in an equal volume of buffer, represented intact cells, fibre associated cells, and microbial biofilms. 0.5 mL of the supernatant and the pellet solution were incubated with 0.5 mL of 30 µmol/L XXXG-resorufin. Xylolglucanase activity was measured as described above. Fecal bacteria from both the enriched diet mice and the control mice exhibited xyloglucanase activity, and more xyloglucase positive CFUs were recovered from the feces of mice fed the xyloglucan-enriched diet indicating that endogenous xyloglucanase activity is increased in the presence of xyloglucan (Figure 19A). Correspondingly, a higher degree of decaging of XXXG-resorufin was observed in the feces from mice fed the xyloglucan-enriched diet (Figure 19B). Moreover, evidence for extracellular endogenous release of endoxyloglucanase was observed.

### EXAMPLE 7: Murine Study of XXXG-PRED and XXXG-DEXA Treatment Efficacy

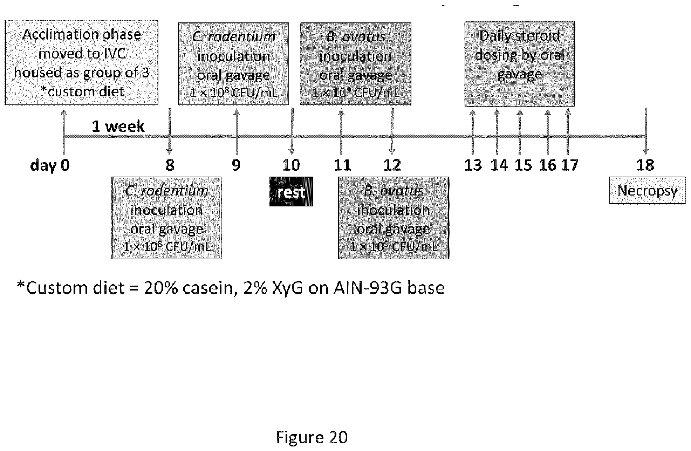

Approval to conduct this project was obtained from the AAFC LeRDC Animal Care Committee (Animal Use Protocol 1911). Similar to Example 3, this study was designed to demonstate the improved efficacy of glyco-caged formulations of prednisolone and dexamethasone over the free forms of prednisolone and dexamethasone during intestinal inflammation. The methods employed were essentially the same as those described in Example 3, except that the *Citrobacter rodentium* (CR) was administered to incite acute self-limiting colitis in mice prior to colonization with *Bacteroides ovatus* (BO). Furthermore, the C57Bl/6 mice used possessed a normal enteric microbiota. Figure 20 provides a schematic illustrating the study design and timelines.

The treatment groups were as summarized below:
- No treatment (CR-/BO+), *n* = 6 mice
- No treatment (CR+/BO+), *n* = 6 mice
- Prenisolone 1 mg/kg (CR+/BO+), *n* = 6 mice
- XXXG-PRED, dose equivalent to prenisolone 1 mg/kg (CR+/BO+), *n* = 6 mice
- Dexamethasone 1.5 mg/kg (CR+/BO+), *n* = 6 mice
- XXXG-DEXA, dose equivalent to dexamethasone 1.5 mg/kg (CR+/BO+), *n* = 6 mice

### Results

Inoculation with CR was again found to successfully incite colitis in mice, as evidenced by internal bleeding from the colon and diarrhea.

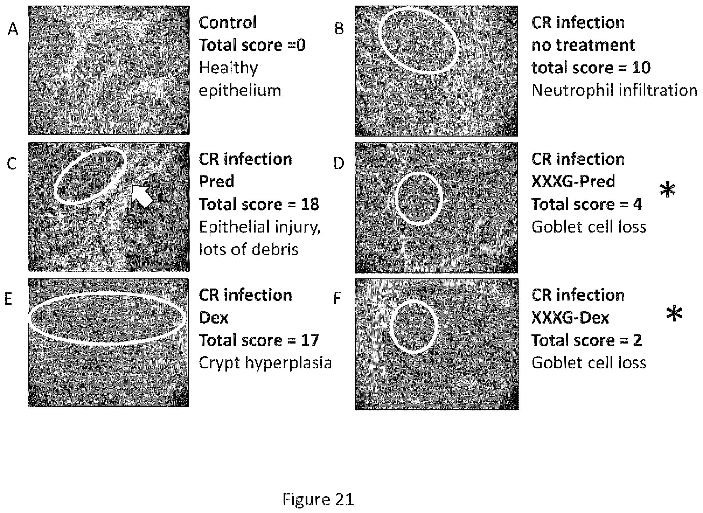

As in the study described in Example 3, there were gross histopathological differences in the distal colon tissue obtained from mice in the different treatment groups. Histological scoring of inflammation was performed by a board-certified pathologist blinded to treatment. Results are shown in Figure 21A-F. Figure 21A shows healthy epithelium from the distal colon of the control group, which was not inoculated with either CR or BO, having a score of 10. Following CR infection, with no treatment, neutrophil infiltration was evident and the histological score was 10. The histological scores were found to be worse following treatment with either prednisolone or dexamethasone (scores 18 and 17, respectively), whereas, treatment with the glyco-caged prodrugs resulted in conspicuous amelioration of inflammation (scores 4 and 2 for XXXG-PRED and XXXG-DEXA, respectively). Evidence of enhanced amelioration of protein markers of inflammation were also observed. For example, quantities of interleukin-12p70 (IL-12p70) protein were significantly lower in colonic tissue of infected female mice administered XXXG-Pred or XXXG-DEXA as compare to infected female mice admistered uncaged PRED or DEXA. IL-12p70 is a pro-inflammatory cytokine involved in cell-mediated immunity. For example, it is known as a T cell-stimulating factor, which can stimulate the growth and function of T cells. Moreover, it stimulates the production of interferon-gamma (IFN-γ) and tumor necrosis factor-alpha (TNF-α) from T cells and natural killer (NK) cells, and reduces IL-4 mediated suppression of IFN-γ.

### Conclusions

Histopathologically, XXXG-DEXA and XXXG-PRED were found to ameliorate colitis incited by *Citrobacter rodentium* in the colon of mice colonized by *Bacteroides ovatus.* XXXG-DEXA exhibited a greater efficacy than uncaged dexamethasone histologically. XXXG-PRED also exhibited a greater efficacy than uncaged prednisolone histologically. Molecular metrics of inflammation supported the histopathologic findings.

## Claims

1. A prodrug compound of formula (I) or (II)
xyloglucan -*β* - O - R^{D} (I)
xyloglucan - *β* - O - L^{SI} - R^{D} (II)
wherein *β* - O represents a *β*-O-glycosidic bond, R^{D} is a drug moiety and L^{SI} is a self-immolative linker moiety,
or a pharmaceutically acceptable salt or solvate thereof,
wherein cleavage of the *β*-O-glycosidic bond connecting the xyloglucan to R^{D} or L^{SI} results in formation of a drug from the drug moiety.

2. The compound according to claim 1, wherein (i) the xyloglucan comprises an XXXG, XXLG, XLXG, or XLLG-type xyloglucan oligosaccharide, or a mixture of two or more of XXXG, XXLG, XLXG, or XLLG-type xyloglucan oligosaccharides, or (ii) the xyloglucan is an XXXG-type xyloglucan oligosaccharide.

3. The compound according to claim 1 or 2, wherein the xyloglucan is obtained from a tamarind seed kernel extract.

4. The compound according to any one of claims 1 to 3, wherein the drug is useful in the treatment of an inflammatory bowel disease, and wherein the drug is optionally (i) an aminosalicylate drug, a corticosteroid, a non-steroidal anti-inflammatory drug, a peptide drug, a natural product, a cannabinoid or an endocannabinoid, or (ii) dexamethasone, prednisolone, budesonide, mesalazine, methyl mesalazine, isofluprednone, meloxicam, flunixin, ketoprofen, larazotide, or cathelicidin KR-12.

5. The compound according to claim 1, which is XXXG-*β*-O-dexamethasone (XXXG-DEXA), XXXG-*β*-O-prednisolone (XXXG-PRED), XXXG-*β*-O-budesonide (BUDE), XXXG-*β*-O-mesalazineME (XXXG-5ASAME), XXXG-*β*-O-mesalazine (XXXG-SASA), or XXXG-SIL-*β*-O-mesalazine (XXXG-SIL-5ASA).

6. A composition comprising the prodrug compound of any one of claims 1 to 5 and a pharmaceutically acceptable diluent or excipient, which is optionally formulated for oral administration.

7. The composition according to claim 6, which additionally comprises one or more pre-biotic polysaccharides, such as one or more xyloglucans, and/or a probiotic, wherein the probiotic optionally comprises:
(i) a *Bacteroidetes* culture, such as a culture of *Bacteroides ovatus, Bacteroides uniformis, Bacteroides cellulosyliticus, Bacteroides faecis, Bacteroides xylanisolvens, Bacteroides fluxus, Bacteroides coprocola,* or *Bacteroides salanitronis,* or a combination thereof; and/or
(ii) a culture of *Bacillus parlicheniformis, Paeniclostridium sordellii, Parabacteroides distansonis, Prevotella* sp., *Salmonella enterica, Streptococcus gallolyticus* (e.g., subsp. *gallolyticus*)*,* or a combination thereof.

8. The compound according to any one of claims 1 to 5 or the composition according to claim 6 or 7, for use as a medicament.

9. The compound or composition according to claim 8, for use in treating a gastrointestinal disorder, such as inflammatory bowel disease, wherein the compound or composition is optionally formulated for oral administration.

10. The compound or composition for use according to claim 9, wherein the compound or composition is for administration with a probiotic, wherein the probiotic optionally comprises:
(i) a *Bacteroidetes* culture, such as a culture of *Bacteroides ovatus, Bacteroides uniformis, Bacteroides cellulosyliticus, Bacteroides faecis, Bacteroides xylanisolvens, Bacteroides fluxus, Bacteroides coprocola,* or *Bacteroides salanitronis,* or a combination thereof; and/or
(ii) a culture of *Bacillus parlicheniformis, Paeniclostridium sordellii, Parabacteroides distansonis, Prevotella* sp., *Salmonella enterica, Streptococcus gallolyticus* (e.g., subsp. *gallolyticus*)*,* or a combination thereof; and
wherein the compound or composition is optionally formulated for administration together with the probiotic or after administration of the probiotic.

11. A method for producing a prodrug compound according to any one of claims 1 to 5, wherein the prodrug compound has the structure of formula (I)
xyloglucan - *β* - O - R^{D} (I)
wherein *β* - O represents a *β*-O-glycosidic bond and R^{D} is a drug moiety,
or a pharmaceutically acceptable salt or solvate thereof,
said method comprising:
(a) protecting the hydroxyl groups of a xyloglucan, optionally by acetylation, to form a protected xyloglucan;
(b) performing a glycosylation reaction to form a glycosidic bond between the protected xyloglucan and a drug or a drug derivative; and
(c) deprotecting the product of step (b).

12. The method of claim 11, wherein the glycosylation reaction of step (b) comprises:
(i) the steps of:
- reacting the protected xyloglucan to form a glycosyl halide, such as a glycosyl bromide; and
- performing a substitution reaction with an alcohol-containing drug or drug derivative and the glycosyl halide; or
(ii) the steps of:
- reacting the protected xyloglucan to form a glycosyl halide;
- treating the glycosyl halide with Ag₂CO₃ to form a glycosyl alcohol;
- treating the glycosyl alcohol with trichloroacetonitrile to form an *O*-glycosyl trichloroacetimidate; and
- performing a substitution reaction with an alcohol-containing drug or drug derivative and the O-glycosyl trichloroacetimidate.

13. The method of claim 11 or 12, wherein the drug derivative is methyl 5-nitrosalicylate or a salt thereof, and wherein optionally prior to step (c), a reduction step is performed to hydrogenate the nitro group of the methyl 5-nitrosalicylate moiety of the product of step (b), or wherein optionally the method additionally comprises:
(d) performing a hydrolysis to remove the methyl group of the methyl 5-nitrosalicylate moiety of the product of step (c), and
(e) performing a reduction step to hydrogenate the nitro group of the 5-nitrosalicylate moiety of the product of step (d).

14. A method for producing a prodrug compound according to any one of claims 1 to 5,
wherein the prodrug compound has the structure of formula (II)
xyloglucan - *β* - O - L^{SI} - R^{D} (II)
wherein *β* - O represents a *β*-O-glycosidic bond, R^{D} is a drug moiety and L^{SI} is a self-immolative linker moiety,
or a pharmaceutically acceptable salt or solvate thereof,
said method comprising:
(a) protecting the hydroxyl groups of a xyloglucan to form a protected xyloglucan;
(b) performing a glycosylation reaction to form a glycosidic bond between the protected xyloglucan and a linker molecule to form a compound of formula (III)
xyloglucan - *β* - O - L^{SI} (III)
wherein the glycosylation reaction optionally comprises:
- reacting the protected xyloglucan to form a glycosyl halide, such as a glycosyl bromide; and
- performing a substitution reaction with the linker molecule or a derivative of the linker molecule and the glycosyl halide; and
(c) attaching a drug or a drug derivative to L^{SI} of the compound of formula (III).

15. The method of claim 14, wherein *β* - O - L^{SI} is a phenyl glycoside/carbamate self-immolative linker, a carbamoyl glycoside self-immolative linker, an alkyl glycoside self-immolative linker, or a phenyl glycoside-benzyl ester self-immolative linker.

## Patentansprüche

1. Prodrug-Verbindung der Formel (I) oder (II)
Xyloglucan-*β*-O-R^{D} (I)
Xyloglucan-*β*-O-L^{SI}- R^{D} (II)
wobei β-0 eine β-O-glykosidische Bindung darstellt, R^{D} ein Arzneimittelanteil ist und L^{SI} ein selbstimmolativer Linkeranteil ist,
oder ein pharmazeutisch annehmbares Salz oder Solvat davon,
wobei die Spaltung der *β*-O-glykosidischen Bindung, die das Xyloglucan mit R^{D} oder L^{SR} verbindet, zur Bildung eines Arzneimittels aus dem Arzneimittelanteil führt.

2. Verbindung nach Anspruch 1, wobei (i) das Xyloglucan ein Xyloglucan-Oligosaccharid vom XXXG-, XXLG-, XLXG- oder XLLG-Typ oder ein Gemisch aus zwei oder mehr Xyloglucan-Oligosacchariden vom XXXG-, XXLG-, XLXG- oder XLLG-Typ umfasst, oder (ii) das Xyloglucan ein Xyloglucan-Oligosaccharid vom XXXG-Typ ist.

3. Verbindung nach Anspruch 1 oder 2, wobei das Xyloglucan aus einem Tamarindensamenkern-Extrakt erhalten wird.

4. Verbindung nach einem der Ansprüche 1 bis 3, wobei das Arzneimittel bei der Behandlung einer entzündlichen Darmerkrankung nützlich ist und wobei das Arzneimittel gegebenenfalls (i) ein Aminosalicylat-Arzneimittel, ein Kortikosteroid, ein nicht-steroidales entzündungshemmendes Arzneimittel, ein Peptid-Arzneimittel, ein Naturprodukt, ein Cannabinoid oder ein Endocannabinoid, oder (ii) Dexamethason, Prednisolon, Budesonid, Mesalazin, Methylmesalazin, Isofluprednon, Meloxicam, Flunixin, Ketoprofen, Larazotid oder Cathelicidin KR-12 ist.

5. Verbindung nach Anspruch 1, die XXXG-*β*-O-Dexamethason (XXXG-DEXA), XXXG-/3-O-Prednisolon (XXXG-PRED), XXXG-*β*-O-Budesonid (BUDE), XXXG-/3-O-MesalazinME (XXXG-5ASAME), XXXG-/3-0-Mesalazin (XXXG-5ASA) oder XXXG-SIL-/3-O-Mesalazin (XXXG-SIL-5ASA) ist.

6. Zusammensetzung, umfassend die Prodrug-Verbindung nach einem der Ansprüche 1 bis 5 und ein pharmazeutisch annehmbares Verdünnungsmittel oder einen pharmazeutisch annehmbaren Hilfsstoff, die gegebenenfalls für die orale Verabreichung formuliert ist.

7. Zusammensetzung nach Anspruch 6, die zusätzlich ein oder mehrere präbiotische Polysaccharide, wie ein oder mehrere Xyloglucane, und/oder ein Probiotikum umfasst, wobei das Probiotikum gegebenenfalls umfasst:
(i) eine *Bacteroidetes*-Kultur, wie eine Kultur von *Bacteroides ovatus, Bacteroides uniformis, Bacteroides cellulosyliticus, Bacteroides faecis, Bacteroides xylanisolvens, Bacteroides fluxus, Bacteroides coprocola* oder *Bacteroides salanitronis,* oder eine Kombination davon; und/oder
(ii) eine Kultur von *Bacillus parlicheniformis, Paeniclostridium sordellii, Parabacteroides distansonis, Prevotella sp., Salmonella enterica, Streptococcus gallolyticus* (z. B. Unterart *gallolyticus)* oder eine Kombination davon.

8. Verbindung nach einem der Ansprüche 1 bis 5 oder die Zusammensetzung nach Anspruch 6 oder 7, zur Verwendung als Medikament.

9. Verbindung oder Zusammensetzung nach Anspruch 8 zur Verwendung bei der Behandlung einer gastrointestinalen Störung, wie z. B. einer entzündlichen Darmerkrankung, wobei die Verbindung oder Zusammensetzung gegebenenfalls für eine orale Verabreichung formuliert ist.

10. Verbindung oder Zusammensetzung zur Verwendung nach Anspruch 9, wobei die Verbindung oder Zusammensetzung zur Verabreichung mit einem Probiotikum bestimmt ist, wobei das Probiotikum gegebenenfalls umfasst:
(i) eine *Bacteroidetes-*Kultur*,* wie z. B. eine Kultur von *Bacteroides ovatus, Bacteroides uniformis, Bacteroides cellulosyliticus, Bacteroides faecis, Bacteroides xylanisolvens, Bacteroides fluxus, Bacteroides coprocola,* oder *Bacteroides salanitronis,* oder eine Kombination davon; und/oder
(ii) eine Kultur von *Bacillus parlicheniformis, Paeniclostridium sordellii, Parabacteroides distansonis, Prevotella-Art, Salmonella enterica, Streptococcus gallolyticus* (z. B. Unterart gallolyticus) oder eine Kombination davon; und
wobei die Verbindung oder Zusammensetzung gegebenenfalls zur Verabreichung zusammen mit dem Probiotikum oder nach Verabreichung des Probiotikums formuliert ist.

11. Verfahren zum Herstellen einer Prodrug-Verbindung nach einem der Ansprüche 1 bis 5, wobei die Prodrug-Verbindung die Struktur der Formel (I) hat
Xyloglucan-*β*-O-R^{D} (I)
wobei β-O eine β-O-glykosidische Bindung darstellt und R^{D} ein Arzneimittelanteil ist,
oder ein pharmazeutisch akzeptables Salz oder Solvat davon,
wobei das Verfahren umfasst:
(a) Schützen der Hydroxylgruppen eines Xyloglucans, gegebenenfalls durch Acetylierung, um ein geschütztes Xyloglucan zu bilden;
(b) Durchführen einer Glykosylierungsreaktion zum Bilden einer glykosidischen Bindung zwischen dem geschützten Xyloglucan und einem Arzneimittel oder einem Arzneimittelderivat; und
(c) Entschützen des Produkts von Schritt (b).

12. Verfahren nach Anspruch 11, wobei die Glykosylierungsreaktion von Schritt (b) umfasst:
(i) die Schritte:
- Reagieren lassen des geschützten Xyloglucans, um ein Glycosylhalogenid zu bilden, wie z. B. ein Glycosylbromid, und
- Durchführen einer Substitutionsreaktion mit einem alkoholhaltigen Arzneimittel oder Arzneimittelderivat und dem Glycosylhalogenid; oder
(ii) die Schritte:
- Reagieren lassen des geschützten Xyloglucans, um ein Glycosylhalogenid zu bilden;
- Behandeln des Glycosylhalogenids mit Ag₂CO₃, um einen Glycosylalkohol zu bilden;
- Behandeln des Glycosylalkohols mit Trichloracetonitril, um ein O-Glycosyltrichloracetimidat zu bilden; und
- Durchführen einer Substitutionsreaktion mit einem alkoholhaltigen Arzneimittel oder Arzneimittelderivat und dem O-Glykosyltrichloracetimidat.

13. Verfahren nach Anspruch 11 oder 12, wobei das Arzneimittelderivat 5-Nitrosalicylat-Methyl oder ein Salz davon ist, und wobei gegebenenfalls vor Schritt (c) ein Reduktionsschritt durchgeführt wird, um die Nitrogruppe des 5-Nitrosalicylat-Methyl-Anteils des Produkts von Schritt (b) zu hydrieren, oder wobei das Verfahren gegebenenfalls zusätzlich umfasst:
(d) Durchführen einer Hydrolyse, um die Methylgruppe des Methyl-5-nitrosalicylat-Anteils des Produkts von Schritt (c) zu entfernen, und
(e) Durchführen eines Reduktionsschritts, um die Nitrogruppe des 5-Nitrosalicylat-Anteils des Produkts von Schritt (d) zu hydrieren.

14. Verfahren zum Herstellen einer Prodrug-Verbindung nach einem der Ansprüche 1 bis 5, wobei die Prodrug-Verbindung die Struktur der Formel (II) hat
Xyloglucan-*β*-O-L^{SI}-R^{D} (II)
wobei β-O eine β-O-glykosidische Bindung darstellt, R^{D} ein Arzneimittelanteil ist und L^{SI} ein selbstimmolativer Linkeranteil ist,
oder ein pharmazeutisch annehmbares Salz oder Solvat davon,
wobei das Verfahren umfasst:
(a) Schützen der Hydroxylgruppen eines Xyloglucans, um ein geschütztes Xyloglucan zu bilden;
(b) Durchführen einer Glykosylierungsreaktion, um eine glykosidische Bindung zwischen dem geschützten Xyloglucan und einem Linkermolekül zu bilden, um eine Verbindung der Formel (III) zu bilden
Xyloglucan-*β*-O-L^{SI} (III)
wobei die Glykosylierungsreaktion gegebenenfalls umfasst:
- Reagieren lassen des geschützten Xyloglucans, um ein Glycosylhalogenid zu bilden, wie z. B. ein Glycosylbromid; und
- Durchführen einer Substitutionsreaktion mit dem Linkermolekül oder einem Derivat des Linkermoleküls und dem Glycosylhalogenid; und
(c) Anbringen eines Arzneimittels oder eines Arzneimittelderivats an L^{SI} der Verbindung der Formel (III).

15. Verfahren nach Anspruch 14, wobei *β*-O-L^{SI} ein selbstimmolativer Linker von Phenylglycosid/Carbamat, ein selbstimmolativer Linker von Carbamoylglycosid, ein selbstimmolativer Linker von Alkylglycosid oder ein selbstimmolativer Linker von Phenylglycosid-Benzylester ist.

## Revendications

1. Composé précurseur de médicament, de formule (I) ou II)
xyloglucane - *β* - O - R^{D} (I)
xyloglucane - *β* - O - L^{SI} - R^{D} (II)
formules dans lesquelles /3 - O représente une liaison *β*-O-glycosidique, R^{D} est un fragment médicamenteux et L^{SI} est un fragment de liaison auto-immolateur,
ou sel ou produit de solvatation pharmaceutiquement acceptables d'un tel composé,
dans lequel le clivage de la liaison *β*-O-glycosidique rattachant le xyloglucane à R^{D} ou L^{SI} conduit à la formation d'un médicament provenant du fragment médicamenteux.

2. Composé selon la revendication 1, dans lequel (i) le xyloglucane comprend un oligosaccharide xyloglucane de type XXXG, XXLG, XLXG, ou XLLG, ou un mélange de deux ou plus de deux des oligosaccharides xyloglucane de type XXXG, XXLG, XLXG, ou XLLG, ou (ii) le xyloglucane est un oligosaccharide xyloglucane de type XXXG.

3. Composé selon la revendication 1 ou 2, dans lequel le xyloglucane est obtenu à partir d'un extrait de graines de tamarinier.

4. Composé selon l'une quelconque des revendications 1 à 3, dans lequel le médicament est utile dans le traitement d'une maladie intestinale inflammatoire, et dans lequel le médicament est en option (i) un médicament aminosalicylate, un corticostéroïde, un médicament anti-inflammatoire non stéroïdien, un médicament peptidique, un produit naturel, un cannabinoïde ou un endocannabinoïde, ou (ii) la dexaméthasone, la prednisolone, le budénoside, la mésalazine, la méthylmésalazine, l'isofluprednone, le méloxicam, la flunixine, le kétoprofène, le larazotide, ou la cathélicidine KR-12.

5. Composé selon la revendication 1, qui est la XXXG-*β*-O-dexaméthasone (XXXG-DEXA), la XXXG-*β*-O-prednisolone (XXXG-PRED), le XXXG-*β*-O-budésonide (BUDE), la XXXG-*β*-O-mésalazineME (XXXG-5ASAME), la XXXG-*β*-O-mésalazine (XXXG-5ASA), ou la XXXG-SIL-*β*-O-mésalazine (XXXG-SIL-5ASA).

6. Composition comprenant le composé précurseur de médicament selon l'une quelconque des revendications 1 à 5 et un diluant ou excipient pharmaceutiquement acceptables, qui est en option formulée pour administration orale.

7. Composition selon la revendication 6, qui comprend en outre un ou plusieurs polysaccharides prébiotiques, tel(s) qu'un ou plusieurs xyloglucanes, et/ou un probiotique, le probiotique comprenant en option :
(i) une culture de *Bacteroidetes,* telle qu'une culture de *Bacteroides ovatus, Bacteroides uniformis, Bacteroides cellulosyliticus, Bacteroides faecis, Bacteroides xylanisolvens, Bacteroides fluxus, Bacteroides coprocola,* ou *Bacteroides salanitronis,* ou une association de telles cultures ; et/ou
(ii) une culture de *Bacillus parlicheniformis, Paeniclostridium sordellii, Parabacteroides distansonis, Prevotella sp., Salmonella enterica, Streptococcus gallolyticus* (par exemple , subsp. *gallolyticus*)*,* ou une association de telles cultures.

8. Composé selon l'une quelconque des revendications 1 à 5 ou composition selon la revendication 6 ou 7, pour utilisation en tant que médicament.

9. Composé ou composition selon la revendication 8, pour utilisation dans le traitement d'un trouble gastro-intestinal, tel qu'une maladie intestinale inflammatoire, le composé ou la composition étant en option formulé(e) pour administration orale.

10. Composé ou composition pour utilisation selon la revendication 9, le composé ou la composition étant pour administration avec un probiotique, le probiotique comprenant en option :
(i) une culture de *Bacteroidetes,* telle qu'une culture de *Bacteroides ovatus, Bacteroides uniformis, Bacteroides cellulosyliticus, Bacteroides faecis, Bacteroides xylanisolvens, Bacteroides fluxus, Bacteroides coprocola,* ou *Bacteroides salanitronis,* ou une association de telles cultures ; et/ou
(ii) une culture de *Bacillus parlicheniformis, Paeniclostridium sordellii, Parabacteroides distansonis, Prevotella sp., Salmonella enterica, Streptococcus gallolyticus* (par exemple , subsp. *gallolyticus*)*,* ou une association de telles cultures ; et
Le composé ou la composition étant en option formulé(e) pour administration conjointement avec le probiotique ou après administration du probiotique.

11. Procédé pour produire un composé précurseur de médicament selon l'une quelconque des revendications 1 à 5, dans lequel le composé précurseur de médicament a la structure de formule (I)
xyloglucane - *β* - O - R^{D} (I)
dans laquelle /3 - O représente une liaison *β*-O-glycosidique et R° est un fragment médicamenteux,
ou un sel ou produit de solvatation pharmaceutiquement acceptables d'un tel composé, ledit procédé comprenant les étapes consistant à :
(a) protéger les groupes hydroxy d'un xyloglucane, en option par acétylation, pour former un xyloglucane protégé ;
(b) effectuer une réaction de glycosylation pour former une liaison glycosidique entre le xyloglucane protégé et un médicament ou un dérivé de médicament, et
(c) déprotéger le produit de l'étape (b).

12. Procédé selon l'étape 11, dans lequel la réaction de glycosylation de l'étape (b) comprend :
(i) les étapes consistant à :
- faire réagir le xyloglucane protégé pour former un halogénure de glycosyle, tel qu'un bromure de glycosyle ; et
- effectuer une réaction de substitution avec un médicament ou dérivé de médicament contenant un alcool et l'halogénure de glycosyle ; ou
(ii) les étapes consistant à :
- faire réagir le xyloglucane protégé pour former un halogénure de glycosyle :
- traiter l'halogénure de glycosyle par Ag₂CO₃ pour former un alcool glycosylique ;
- traiter l'alcool glycosylique par du trichloroacétonitrile pour former un trichloroacétimidate d'O-glycosyle ; et
- effectuer une réaction de substitution avec un médicament ou dérivé de médicament contenant un alcool et le trichloroacétimidate d'O-glycosyle.

13. Procédé selon la revendication 11 ou 12, dans lequel le dérivé de médicament est le 5-nitrosalicylate de méthyle ou un sel de celui-ci, et dans lequel en option avant l'étape (c), une étape de réduction est effectuée pour hydrogéner le groupe nitro du fragment 5-nitrosalicylate de méthyle du produit de l'étape (b), ou dans lequel en option le procédé comprend en outre :
(d) effectuer une hydrolyse pour éliminer le groupe méthyle du fragment 5-nitrosalicylate de méthyle du produit de l'étape (c), et
(e) effectuer une étape de réduction pour hydrogéner le groupe nitro du fragment 5-nitrosalicylate du produit de l'étape (d).

14. Procédé pour produire un composé précurseur de médicament selon l'une quelconque des revendications 1 à 5, dans lequel le composé précurseur de médicament a la structure de formule (II)
xyloglucane - *β* - O - L^{SI} - R^{D} (II)
dans laquelle /3 - O représente une liaison *β*-O-glycosidique et R^{D} est un fragment médicamenteux et L^{SI} est un fragment de liaison auto-immolateur,
ou un sel ou produit de solvatation pharmaceutiquement acceptables d'un tel composé, ledit procédé comprenant les étapes consistant à :
(a) protéger les groupes hydroxy d'un xyloglucane pour former un xyloglucane protégé ;
(b) effectuer une réaction de glycosylation pour former une liaison glycosidique entre le xyloglucane protégé et une molécule de liaison pour former un composé de formule (III)
xyloglucane - *β* - O - L^{SI} (III)
la réaction de glycosylation comprenant en option :
- faire réagir le xyloglucane protégé pour former un halogénure de glycosyle, tel qu'un bromure de glycosyle, et
- effectuer une réaction de substitution avec la molécule de liaison ou un dérivé de la molécule de liaison et l'halogénure de glycosyle ; et
(c) attacher un médicament ou un dérivé de médicament à L^{SI} du composé de formule (III).

15. Procédé selon la revendication 14, dans lequel /3 - O - L^{SI} est un groupement de liaison auto-immolateur phényl glycoside/carbamate, un groupement de liaison auto-immolateur carbamoyl glycoside, un groupement de liaison auto-immolateur alkyl glycoside, ou un groupement de liaison auto-immolateur phényl glycoside/ester benzylique.
